(19) Europäisches Patentamt / European Patent Office / Office européen des brevets

(11) **EP 2 170 158 B1**

(12) **EUROPEAN PATENT SPECIFICATION**

(45) Date of publication and mention
of the grant of the patent:
**05.07.2017 Bulletin 2017/27**

(21) Application number: **08769447.7**

(22) Date of filing: **12.05.2008**

(51) Int Cl.:
*A61B 5/00* (2006.01)  *G06F 19/00* (2011.01)

(86) International application number:
**PCT/US2008/063402**

(87) International publication number:
**WO 2009/002622 (31.12.2008 Gazette 2009/01)**

(54) **PATIENT INFORMATION INPUT INTERFACE FOR A THERAPY SYSTEM**

SCHNITTSTELLE ZUR EINGABE VON PATIENTENDATEN FÜR EIN THERAPIESYSTEM

INTERFACE D'ENTRÉE D'INFORMATIONS DE PATIENT POUR UN SYSTÈME DE THÉRAPIE

(84) Designated Contracting States:
**AT BE BG CH CY CZ DE DK EE ES FI FR GB GR
HR HU IE IS IT LI LT LU LV MC MT NL NO PL PT
RO SE SI SK TR**

(30) Priority: **27.06.2007 US 946606 P**

(43) Date of publication of application:
**07.04.2010 Bulletin 2010/14**

(73) Proprietors:
• **F. Hoffmann-La Roche AG**
**4070 Basel (CH)**
Designated Contracting States:
**AT BE BG CH CY CZ DK EE ES FI FR GB GR HR
HU IE IS IT LI LT LU LV MC MT NL NO PL PT RO
SE SI SK TR**
• **Roche Diabetes Care GmbH**
**68305 Mannheim (DE)**
Designated Contracting States:
**DE**

(72) Inventors:
• **WEINERT, Stefan**
**Pendleton, IN 46064 (US)**
• **THUKRAL, Ajay**
**Fishers, IN 46037 (US)**

(56) References cited:
EP-A- 1 580 682    WO-A-03/063684
WO-A-2007/065285    US-A1- 2005 197 621

• **BELLAZZI R ET AL: "THE SUBCUTANEOUS
ROUTE TO INSULIN-DEPENDENT DIABETES
THERAPY - CLOSED-LOOP AND PARTIALLY
CLOSED-LOOP CONTROL STRATEGIES FOR
INSULIN DELIVERY AND MEASURING GLUCOSE
CONCENTRATION" IEEE ENGINEERING IN
MEDICINE AND BIOLOGY MAGAZINE, IEEE
SERVICE CENTER, PISACATAWAY, NJ, US, vol.
20, no. 1, 1 January 2001 (2001-01-01), pages
54-64, XP011085089 ISSN: 0739-5175**

EP 2 170 158 B1

**Description**

The present invention relates generally to techniques for determining therapy information, such as drug and/or other therapy information, and more specifically to systems for determining such therapy information based on patient input of information that characterizes at least one patient event or condition. An exemplary system is known from WO 03/063684 A2.

A number of drug control arrangements exist that are configured to recommend or automatically administer drug therapy, based on some amount of information provided by the patient. It is desirable to provide a patient-specific interface that the patient may routinely use to supply information that characterizes at least one patient event or condition and that uses the supplied information to determine corresponding drug and/or other therapy.

The present invention is defined in claims 1 and 14 and may comprise one or more of the features recited in the attached dependent claims. A method is provided for developing a patient interface for a therapy system that the patient may use to input information that characterizes at least one patient event or condition and from which therapy information can be determined. The method may comprise developing a patient model that is configured to simulate the patient's physiological response to the at least one patient event or condition, collecting patient-specific information over time that relates to actual occurrences of the at least one patient event or condition, and defining a graphical interface, based on the patient model and on the collected patient-specific information, that maps input from the patient of the information that characterizes the at least one patient event or condition to corresponding therapy information.

The therapy information may comprise drug therapy information corresponding to one or more drugs to be administered to the patient at some time. The administering time may be, for example, a current time, a future time, times determined by the analysis, times determined by the end-user, a time window, and the likes. Alternatively or additionally, the therapy information may comprise suggested carbohydrate intake information corresponding to a recommendation to ingest carbohydrates. Alternatively or additionally, the therapy information may comprise suggested exercise information corresponding to a recommendation to undertake exercise. Alternatively or additionally, the therapy information may comprise a recommendation to consult a physician.

Defining a graphical interface may comprise selecting a graphical interface having two input parameters that characterize the at least one patient event or condition. Defining a graphical interface may further comprise defining a solution space of the selected graphical interface based on the patient model and on the collected patient-specific information. Defining a graphical interface may further comprise defining a map that maps the two input parameters that characterize the at least one patient event or condition to the corresponding therapy information.

The method may further comprise using graphical interface to map patient input of the information that characterizes the at least one patient event or condition to corresponding therapy information. The corresponding therapy information may comprise drug administration information. The method may further comprise displaying the drug administration information in the form of a recommended dosage of the drug. Alternatively or additionally, the method may further comprise controlling a drug administration device to administer to the patient at least one amount of the drug based on the drug administration information.

In another embodiment, a method of developing a patient interface for a therapy system that a patient may use to input information which characterizes at least one patient event or condition and from which therapy information can be determined is disclosed. The method comprises receiving patient specific information having input parameters characterizes the at least one patient event or condition; identifying which of the input parameters have discrepancies to a corresponding predetermined value provided in a predetermined therapy information; setting up a constrained minimization problem for the identified input parameters; generating a solution space from solving the constrained minimization problem, wherein the solution space defines a relationship between the input parameters and their associated acceptable limits to regulate a patient's physiological response to a desired target response; and implementing the solution space as the patient interface.

A system for developing a patient interface for a therapy system that the patient may use to input information that characterizes at least one patient event or condition and from which therapy information can be determined may comprise a database having stored therein a patient model that is configured to simulate the patient's physiological response to the at least one patient event or condition, a first memory configured to store therein patient-specific information over time that relates to actual occurrences of the at least one patient event or condition, and a graphical interface that is configured to map, based on the patient model and on the collected patient-specific information, input from the patient of the information that characterizes the at least one patient event or condition to corresponding therapy information.

The system may further comprise a processor having access to a second memory that has stored therein instructions that are executable by the processor to process the input from the patient to the graphical interface of the information that characterizes the at least one patient and produce the corresponding therapy information. The system may further comprise a display unit. The second memory further may have stored therein instructions that are executable by the processor to control the display unit to display the corresponding therapy information. The system may further comprise a manually actuatable drug administration device. The corresponding therapy information may comprise at least one drug quantity. The second memory may further have stored therein instructions that are executable by the processor to control the display unit to display the at least one drug quantity that may be administered by the patient using the manually actuatable drug administration device. The system may further comprise a blood glucose sensor configured to measure a blood glucose level of the patient and produce a corresponding blood glucose value. The second memory may further have stored therein instructions that are executable by the processor to determine the at least one drug quantity further based on the blood glucose value.

Alternatively or additionally, the system may further comprise an electronically controllable drug administration device configured to administer at least one drug to the patient. The corresponding therapy information may comprise at least one drug quantity in one embodiment, and in another embodiment, a distributed sequence of a drug determined by time and amount. The second memory may further have stored therein instructions that are executable by the processor to control the electronically controllable drug administration device to administer the at least one drug quantity to the patient. The system may further comprise a blood glucose sensor configured to measure a blood glucose level of the patient and produce a corresponding blood glucose value. The second memory may further have stored therein instructions that are executable by the processor to determine the at least one drug quantity further based on the blood glucose value.

The graphical interface may be configured to map patient input of at least two parameters that characterize the at least one patient event or condition to the corresponding therapy information.

FIG. 1 is a block diagram of one illustrative embodiment of a system for determining therapy information.

FIG. 2 is a flowchart of one illustrative process for developing a patient interface that may be used with the system of FIG. 1 to allow a patient to input patient-related information from which therapy information can be determined.

FIG. 3 is a diagram showing possible components of one exemplary patient-specific gluco-regulatory model that may be configured to allow for the input of patient-specific information that characterizes at least one patient-related event or condition.

FIG. 4 is a block diagram illustration of a compartmental model of arterial inflow and venous outflow that is used to develop a gluco-regulatory model to simulate glucose concentration and the distribution of insulin in various organs and body regions.

FIG. 5 is a schematic of the gluco-regulatory model of FIG. 3, constructed using several of the compartmental models of FIG. 4, to simulate glucose concentration in the various organs and areas of the body.

FIG. 6 is a schematic of the of the gluco-regulatory model of FIG. 3, constructed using several of the compartmental models of FIG. 4, to simulate insulin kinetics in the various organs and areas of the body.

FIG. 7 illustrates one embodiment of a graphical patient interface that may be used to enter meal-related information into the system of FIG. 1.

FIG. 8 illustrates another embodiment of a graphical patient interface that may be used to enter meal-related information into the system of FIG. 1.

FIG. 9 illustrates a further embodiment of a graphical patient interface that may be used to enter meal-related information into the system of FIG. 1.

FIG. 10 illustrates yet another embodiment of a graphical patient interface that may be used to enter meal-related information into the system of FIG. 1.

FIG. 11 illustrates still another embodiment of a graphical patient interface that may be used to enter meal-related information into the system of FIG. 1.

FIG. 12 illustrates yet a further embodiment of a graphical patient interface that may be used to enter meal-related information into the system of FIG. 1.

FIG. 13 is a plot of meal space input parameters illustrating the formation of the outer periphery of an example graphical interface.

FIG. 14 is a plot of insulin bolus quantity vs. the total meal space $\eta^{A/S}$ illustrating the solution space resulting from the formation of the outer periphery of the example graphical interface of FIG. 13.

FIG. 15 a plot of one of the meal space parameters vs. a number of discrete user inputs.

FIG. 16 is a table illustrating one embodiment of a map correlating patient input of meal information, provided in the form of carbohydrate content, e.g., meal size, and expected glucose absorption shape and duration, e.g., meal duration, to corresponding drug therapy information.

FIG. 17 is a block diagram illustrating the system of FIG. 1 implemented in a semi-closed loop drug administration system.

FIG. 18 is a flowchart illustrating one embodiment of a software algorithm, executable by the system of FIG. 1, for determining drug therapy information based on user input of meal information using one of the graphical patient interfaces of FIGS. 7-12.

For the purposes of promoting an understanding of the principles of the invention, reference will now be made to a number of illustrative embodiments shown in the attached drawings and specific language will be used to describe the same.

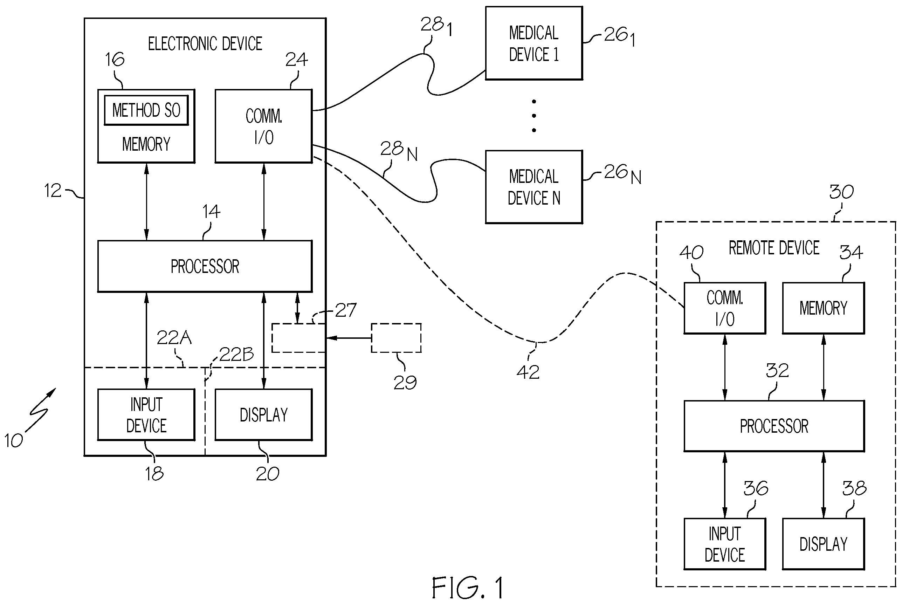

Referring now to FIG. 1, a block diagram of one illustrative embodiment of a system 10 for determining therapy information is shown. In the illustrated embodiment, the system 10 includes an electronic device 12 having a processor 14 in data communication with a memory unit 16, an input device 18, a display 20 and a communication input/output unit 24. The electronic device 12 may be provided in the form of a general purpose computer, central server, personal computer (PC), lap top or notebook computer, personal data assistant (PDA) or other hand-held device, external infusion pump, blood glucose meter, analyte sensing system, or the like. The electronic device 12 may be configured to operate in accordance with one or more conventional operating systems including for example, but not limited to, windows, linux and Mac OS and embedded OS such as QNX, eCOS, WinCE and palm OS, and may be configured to process data according to one or more conventional internet protocols for example, but not limited to, NetBios, TCP/IP and AppleTalk. The processor 14 is, in the illustrated embodiment, microprocessor-based, although the processor 14 may alternatively be formed of one or more general purpose and/or application specific circuits and operable as described hereinafter. The memory unit 16 includes, in the illustrated embodiment, sufficient capacity to store data, one or more software algorithms executable by the processor 14 and other data. The memory unit 16 may include one or more conventional memory or other data storage devices. Alternatively or additionally, the system 10 may include a U3 smart USB device having sufficient capacity to store data and one or more software algorithms executable by the processor 14.

The input device 18 may be used in a conventional manner to input and/or modify data. In the illustrated embodiment, the display 20 is also included for viewing information relating to operation of the device 12 and/or system 10. Such a display may be a conventional display device including for example, but not limited to, a light emitting diode (LED) display, a liquid crystal display (LCD), a cathode ray tube (CRT) display, or the like. Alternatively or additionally, the display 20 may be or include an audible display configured to communicate information to a user, another person or another electronic system having audio recognition capabilities via one or more coded patterns, vibrations, synthesized voice responses, or the like. Alternatively or additionally, the display 20 may be or include one or more tactile indicators configured to display tactile information that may be discerned by the user or another person. A camera may also be included for capturing and storing meal photos and/or other photos.

In one embodiment, the input device 18 may be or include a conventional keyboard or key pad for entering alphanumeric data into the processor 14. Such a keyboard or key pad may include one or more keys or buttons configured with one or more tactile indicators to allow users with poor eyesight to find and select an appropriate one or more of the keys, and/or to allow users to find and select an appropriate one or more of the keys in poor lighting conditions. Alternatively or additionally, the input device 18 may be or include a conventional mouse or other conventional point and click device for selecting information presented on the display 20. Alternatively or additionally, the input device 18 may include the display 20 configured as a graphical user interface (GUI). In this embodiment, the display 20 may include one or more selectable inputs that a user may select by touching an appropriate portion of the display 20 using an appropriate implement. Alternatively or additionally, the input device 18 may include a number of switches or buttons that may be activated by a user to select corresponding operational features of the device 12 and/or system 10. Alternatively or additionally, the input device 18 may be or include voice activated circuitry responsive to voice commands to provide corresponding input data to the processor 14. In any case, the input device 18 and/or display 20 may be included with or separate from the electronic device 12 as indicated by the dashed lines 22A and 22B.

In one or more embodiments, the system 10 may include a number, N, of medical devices $26_1$ - $26_N$, wherein N may be any positive integer. In such embodiments, any of the one or more medical devices $26_1$ - $26_N$ may be implanted within the patient's body, coupled externally to the patient's body (e.g., such as an infusion pump), or be separate and remote from the patient's body. Alternatively or additionally, one or more of the medical devices $26_1$-$26_N$ may be mounted to and/or form part of the electronic device 12. In the illustrated embodiment, the number of medical devices $26_1$ - $26_N$ is each configured to communicate wirelessly with the communication I/O unit 24 of the electronic device 12 via one of a corresponding number of wireless communication links $28_1$ - $28_N$. The wireless communications may be one-way or two-way. The form of wireless communication used may include, but should not be limited to, radio frequency (RF) communication, infrared (IR) communication, WiFi, RFID (inductive coupling) communication, acoustic communication, capacitive signaling (through a conductive body), galvanic signaling (through a conductive body), or the like. In any such case, the electronic device 12 and each of the number of medical devices $26_1$ - $26_N$ include conventional circuitry for conducting such wireless communications circuit. Alternatively or additionally, one or more of the medical devices $26_1$ - $26_N$ may be configured to communicate with the electronic device 12 via one or more conventional serial or parallel configured hardwire connections therebetween and/or via one or more other conventional communication hardware, software and/or firmware.

Each of the one or more medical devices $26_1$ - $26_N$ may include any one or more of a conventional processing unit, conventional input/output circuitry and/or devices and one or more suitable data and/or program storage devices. Examples of the one or more medical devices $26_1$ - $26_N$ include, but are not limited to, one or more blood glucose sensors, one or more body temperature sensors, one or more drug infusion devices, or the like. In one or more embodiments, either in addition to or in lieu of the one or more medical devices $26_1$ - $26_N$, the electronic device 12 may include an on-board analyte sensor in the form of a conventional strip reader 27 that is configured to receive a conventional strip or carrier 29. In this embodiment, the strip or carrier 29 is configured to receive a sample of blood or other bodily fluid and to be inserted into the strip reader 27. The strip reader 27 is electrically connected to the processor 14, and the processor 14 is operable, under the control of one or more software algorithms stored in the memory 16, to process electrical signals produced by the strip reader 27 to determine at least one characteristic of an analyte contained in the fluid that was received on the strip or carrier 29. Illustratively, the fluid may be blood, the analyte may be blood glucose, and the at least one characteristic of the analyte may include a concentration of glucose in the blood. In this embodiment, the analyte sensor is a blood glucose sensor provided in the form of a conventional blood glucose strip reader 27. The blood glucose sensor, in this embodiment, may be a conventional electro-chemical or photometric sensor. It will be understood, however, that the analyte sensor may alternatively be configured to analyze other fluids, analytes and/or analyte characteristics. Any of the one or more medical devices $26_1$ - $26_N$ may include smart cards or biometrics that carry security information and/or relevant medical records.

In some embodiments, the system 10 may alternatively or additionally include a remote device 30, as illustrated in phantom in FIG. 1. The remote device 30 may include a conventional processor 32, which may be identical or similar to the processor 14, a conventional memory or other data storage unit 34, a conventional input device 36, which may be or include any one or more of the input devices described hereinabove with respect to the input device 18, a conventional display unit 38, which may be or include any one or more of the display units described hereinabove with respect to the display unit 20, and conventional communication I/O circuitry 40. The remote device 30 may be configured to communicate with the electronic device 12 via any conventional wired or wireless communication interface 42, which may be or include any of the communication interfaces or links described hereinabove.

Throughout this document, examples of various structures, processes and techniques are provided to illustrate and describe concepts of this disclosure. For consistency, these examples all relate generally to a diabetes control arrangement in which one or more recommended or automatically administered bolus(es) of a glucose-lowering drug is the illustrated and described mechanism for diabetes control, and relate more specifically to meal-related information as the illustrated and described patient input information from which the one or more recommended or automatically administered insulin bolus(es) is/are determined. It will be understood that such examples are provided only for illustrative purposes, and should not be considered to be limiting in any way. Rather, it should be understood that this disclosure relates to any therapy system in which administration of one or more drugs represents only one form of therapy, and that other forms of therapy may alternatively or additionally be determined and recommended in accordance with this disclosure. Examples of other such forms of therapy may include, but are not limited to, recommending exercise, recommending intake of food, e.g., carbohydrates, recommending consult with and/or a visit to a physician, and the like. It should further be understood that in the context of a diabetes control system, this disclosure contemplates that the recommendation or automatic administration of blood glucose lowering medication may be based on patient input of one or more patient-related events and/or conditions other than, or in addition to, meal related information. Other examples include, but are not limited to, patient input that characterizes or otherwise acknowledges the occurrence of patient exercise information, patient illness information, patient-related stress information and the like.

In one or more exemplary embodiments, the therapy system 10 of FIG. 1 may be, or form part of, a conventional fully closed-loop, semi closed-loop or open-loop diabetes control arrangement. In this embodiment, the system 10 provides for patient input of some amount of feed forward information from which the system 10 determines, at least in part, therapy information in the form of insulin bolus administration information and/or other therapy information. Such insulin bolus administration information may be or include, for example, an insulin bolus quantity or quantities, bolus type (e.g., normal or fast-acting, e.g., Regular, Lispro, etc.), insulin bolus delivery time, times or intervals (e.g., single delivery, multiple discrete deliveries, continuous delivery, etc.), and the like. Examples of patient-supplied feed forward information may be or include, but should not be limited to, one or more of patient blood glucose concentration, information relating to carbohydrates in the form of a meal, snack or other form that has been ingested, is being ingested, or is to be ingested sometime in the near future, patient exercise information, patient stress information, patient illness information, information relating to the patient's menstrual cycle, and the like. In any case, the system 10 may include a conventional drug delivery mechanism for administering an amount or amounts of a drug; e.g., insulin, glucagon, incretin, or the like at one or more time instances. Alternatively or additionally, the system 10 may be configured to offer an alternatively actionable therapy recommendation to the user via the display 20, e.g., ingesting carbohydrates, exercising, consulting a physician, adjust and/or take additional or different medication (time and/or quantity), etc. Embodiments of the system 10 that are, or form part of, a conventional fully closed-loop, semi closed-loop or open-loop diabetes control arrangement may be provided in any of a variety of conventional configurations, and examples of some such configurations will now be described. It will be understood, however, that the following examples are provided merely for illustrative purposes, and should not be considered limiting in any way. Those skilled in the art may recognize other possible implementations of a fully closed-loop, semi closed-loop or open-loop diabetes control arrangement, and any such other implementations are contemplated by this disclosure.

In a first specific example implementation of the system 10 in the form of a diabetes control system, the electronic device 12 is provided in the form of a conventional insulin pump configured to be worn externally to the user's body and also configured to controllably deliver insulin to the patient's body. In this example, the number of medical devices $26_1$ - $26_N$ may include one or more implanted sensors configured to provide information relating to the physiological condition of the patient. Examples of such implanted sensors may include, but should not be limited to, a glucose sensor, a body temperature sensor, a blood pressure sensor, a heart rate sensor, one or more bio-markers configured to capture one or more physiological states of the body, e.g., HBA1C, or the like. In implementations that include an implanted glucose sensor, the system 10 may be a fully closed-loop system operable in a conventional manner to automatically monitor blood glucose and deliver insulin, as appropriate, to maintain blood glucose at desired levels. The number of medical devices $26_1$ - $26_N$ may alternatively or additionally include one or more sensors or sensing systems that are external to the user's body and/or sensor techniques for providing information relating to the physiological condition of the user. Alternatively or additionally, the electronic device 12 may include an on-board blood glucose meter in the form of, for example, a conventional blood glucose strip reader 27 as illustrated in phantom in FIG. 1. In any case, examples of such sensors or sensing systems may include, but should not be limited to, a glucose strip sensor/meter, a body temperature sensor, a blood pressure sensor, a heart rate sensor, one or more bio-markers configured to capture one or more physiological states of the body, e.g., HBA1C, or the like. In implementations that include an external glucose sensor, the system 10 may be a closed-loop, semi closed-loop or open-loop system operable in a conventional manner to deliver insulin, as appropriate, based on glucose information provided thereto by the patient. Information provided by any such sensors and/or sensor techniques may be communicated to the system 10 using any one or more conventional wired or wireless communication techniques. In this example implementation, the remote device 30 may also be included in the form of a handheld or otherwise portable electronic device configured to communicate information to and/or from the electronic device 12. In addition and/or in other embodiments, information providing dosing, timing and other information for particular use cases may be used. In one embodiment, such use case information is captured, determined, and provided by another system, such as for example, disclosed by commonly assigned and co-pending PCT Application WO 2009/002621 A2, entitled "MEDICAL DIAGNOSIS, THERAPY, AND PROGNOSIS SYSTEM FOR INVOKED EVENTS AND METHOD THEREOF":

In a second specific example implementation of the system 10 in the form of a diabetes control system, the electronic device 12 is provided in the form of a handheld remote device, such as a PDA or other handheld device. In this example, the number of medical devices $26_1$ - $26_N$ includes at least one conventional implantable or externally worn drug pump. In one embodiment of this example, an insulin pump is configured to controllably deliver insulin to the user's body. In this embodiment, the insulin pump is configured to wirelessly transmit information relating to insulin delivery to the handheld device 12. The handheld device 12 is configured to monitor insulin delivery by the pump, and may further be configured to determine and recommend insulin bolus amounts, carbohydrate intake, exercise, and the like. The system 10 may or may not be configured in this embodiment to provide for transmission of wireless information from the handheld device 12 to the insulin pump. The electronic device 12, in this embodiment, may or may not include an on-board blood glucose meter in the form of, for example, a conventional blood glucose strip reader 27 as illustrated in phantom in FIG. 1.

In an alternate embodiment of this example, the handheld device 12 is configured to control insulin delivery to the user by determining insulin delivery commands and transmitting such commands to the insulin pump. The insulin pump, in turn, is configured to receive the insulin delivery commands from the handheld device 12, and to deliver insulin to the user according to the commands. The insulin pump, in this embodiment, may or may not further process the insulin pump commands provided by the handheld unit 12. In any case, the system 10 will typically be configured in this embodiment to provide for transmission of wireless information from the insulin pump back to the handheld device 12 to thereby allow for monitoring of pump operation. In either embodiment of this example, the system 10 may further include one or more implanted and/or external sensors of the type described in the previous example. In this example implementation, the remote device 30 may also be included in the form of, for example, a PC, PDA, laptop or notebook computer configured to communicate information to and/or from the electronic device 12.

Those skilled in the art will recognize other possible implementations of a fully closed-loop, semi closed-loop or open-loop diabetes control arrangement using at least some of the components of the system 10 illustrated in FIG. 1. For example, the electronic device 12 in one or more of the above examples may be provided in the form of a PDA, laptop, notebook or personal computer configured to communicate with one or more of the medical devices $26_1$ - $26_N$, at least one of which is an insulin delivery system, to monitor and/or control the delivery of insulin to the user. As another example, the remote device 30 may be configured to communicate with the electronic device 12 and/or one or more of the medical devices $26_1$ - $26_N$, to control and/or monitor insulin delivery to the patient, and/or to transfer one or more software programs and/or data to the electronic device 12. The remote device 30 may reside in a caregiver's office or other remote location, and communication between the remote device and any component of the system 10 may be accomplished via an intranet, internet (e.g., world-wide-web), cellular, telephone modem, RF, or other communication link. Any one or more conventional internet protocols may be used in such communications. Alternatively or additionally, any conventional mobile content delivery system; e.g., Wi-Fi, WiMAX, short message system (SMS), or other conventional message schema may be used to provide for communication between devices comprising the system 10. In any case, any such other implementations are contemplated by this disclosure. Alternatively or additionally, the drug delivery mechanism may take the form of one or more of a conventional implantable or externally worn drug infusion mechanism, a conventional drug injection mechanism, such as a drug injection pen or hypodermic needle, a conventional inhalation mechanism for administering one or more inhalable forms of one or more drugs, or the like.

The therapy system 10 of FIG. 1 is generally operable to determine and recommend and/or administer therapy in the form of an appropriate amount of one or more drugs and time, and/or to determine and recommend alternate therapy or action. In determining any such therapy, the system 10 requires at least some information relating to one or more external influences that the patient is subjected to and/or one or more physiological mechanisms associated with the patient. The one or more external influences may be characterized as being action that is typically voluntarily undertaken by the patient, and may be referred to herein as one or more "patient events." In the context of a diabetes control system, examples of such patient events include, but are not limited to, ingesting of carbohydrates, undertaking physical exercise and the like. In contrast, the one or more physiological mechanisms may be characterized as being involuntary bodily states or reactions typically associated with the patient's physiology and/or environment, and may be referred to herein as one or more "patient conditions" or metabolic states In the context of a diabetes control system, examples of such patient conditions include, but are not limited to, illness, stress, menstruation, and the like. In any case, if the patient is about to experience, is experiencing, or has recently experienced one or more patient events and/or conditions, the system 10 generally requires some information relating to at least one of the patient events or conditions to determine an appropriate therapy. In the context of a glucose control system, example therapies may include, but are not limited to, a recommendation or administration of some quantity, type, and/or frequency of a glucose-lowering drug, e.g., insulin, recommendation of carbohydrate ingesting, recommendation of one or more exercises, recommendation to consult with and/or visit a physician, and the like.

The system 10 illustratively includes a graphical interface that is configured to provide for patient (sometimes referred to herein as "user") input in a form that characterizes at least one patient event or condition that may result in the recommendation and/or administration of one or more drugs or other therapy. The graphical interface is illustratively displayed on the display unit 20 of the electronic device 12, but may alternatively or additionally be displayed on the display unit 38 of the remote device 30. The processor 14 is configured to control the display unit 20 to display the graphical interface on the electronic device 12 in a conventional manner. Alternatively or additionally, the processor 32 may be configured to control the display unit 38 to display the graphical interface on the remote device 30 in a conventional manner. User input to the graphical interface may be provided in any one or more conventional forms. Examples include, but are not limited to, one or more buttons or keys provided on the input device 18 and/or 36 of the corresponding device 12 and/or 30, a touch-sensitive screen of the display unit 20 and/or 38, one or more conventional point and click mechanisms, or the like.

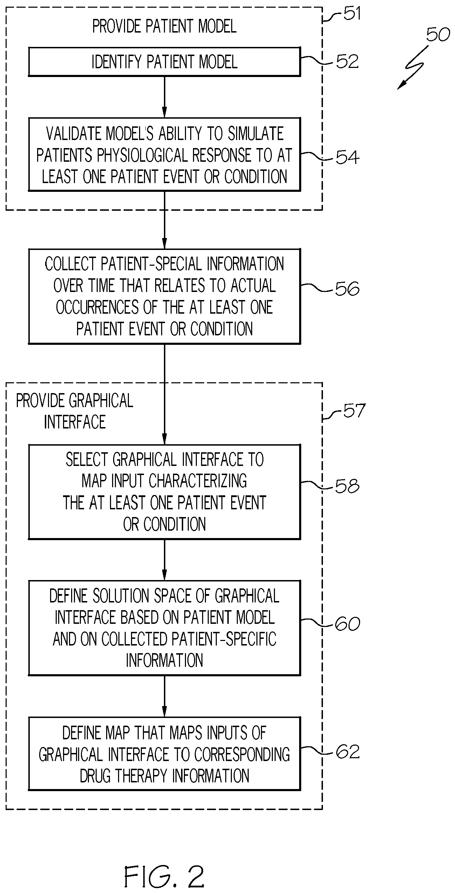

Referring to FIG. 2, a flowchart of one illustrative process 50 is shown for developing a patient interface that may be used with the system 10 of FIG. 1 to allow a patient to input patient-related information from which therapy information can be determined. It is anticipated that the process 50 will typically be conducted by a physician or other health care professional, although this disclosure contemplates that the process 50 may alternatively be conducted by other parties, one example of which may be, but should not be limited to, companies or other entities specializing in disease or illness care or therapy. It is also anticipated that at least some of the process 50 will be carried out with the assistance of at least one computer, and that the process 50 may be carried out with the assistance of a number of different computers wherein at least some of the number of computers have access to one or more databases. Examples of such computer or computers may include, but should not be limited to, the electronic device 12, the electronic device 30, a conventional networked or non-networked personal, laptop or notebook computer that may or may not have access to one or more remote databases via an internet, intranet or similar connection, a conventional mainframe computer, or the like. It is further anticipated that the process 50 will be carried out on a patient-by-patient basis so that the resulting graphical interface will be patient specific.

The process 50 begins at step 52 where a patient model is identified that is suitable for modeling the patient's physiological response to at least one patient event or condition upon which the graphical interface will be based. In one embodiment, the patient model may be determined using the system described in commonly assigned and co-pending PCT ApplicationWO 2009/002620 A1, entitled "SYSTEM FOR DEVELOPING PATIENT-SPECIFIC THERAPIES BASED ON DYNAMIC MODELING OF PATIENT PHYSIOLOGY AND METHOD THEREOF". In the illustrated embodiment, step 52 focuses on determining the structure and parameters of a patient's physiology from a given set of patient-specific data, and is carried out by incorporating modeling concepts that are tied to specific therapy solutions, e.g., drug administration and/or other therapy solutions. This approach may be simplified by considering only a limited set of dynamic patient events or conditions that may affect the model.

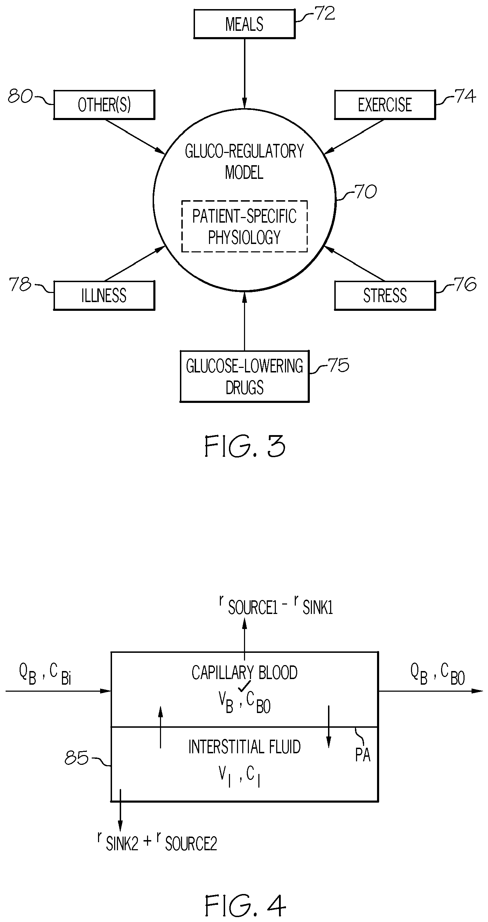

Referring to FIG. 3, a patient modeling framework is illustrated in the context of an example diabetes control arrangement in which the patient model is identified as a conventional gluco-regulatory model 70. The gluco-regulatory model 70 is generally one that simulates human response to blood glucose affecting events or conditions, and the generalized gluco-regulatory model 70 becomes patient-specific by tailoring the model 70 with information that corresponds to patient-specific physiology as shown if FIG. 3 by the dashed-line block within the perimeter of the model 70. The gluco-regulatory model 70 is simplified by considering only a limited number of dynamic patient events or conditions that may affect the state of the model 70, and examples of such dynamic patient events or conditions are illustrated in FIG. 3. These include, but should not be limited to, intake of meals 72, snacks, etc. (e.g., ingesting carbohydrates), exercise 74, patient stress 76, administering one or more glucose lowering drugs 75, patient illness 78, and one or more other patient events or conditions 80. In the simplest case, the gluco-regulatory model 70 is configured to consider only one of the dynamic patient events or conditions, e.g., meals 72, as input information, and model complexity increases as additional dynamic patient events or conditions are considered.

Generally, step 52 captures the structure of the model, i.e., the dynamics or principles of model operation, and identifies model parameters that are specific to the individual patient and/or that define at least one particular patient event or condition. Patient-specific data is collected (as per protocol), as part of step 52, from which such structure and parameters of the patient model are determined and defined. In this manner, the patient model is tailored to the physiology of the individual patient. Additional resources exist for identifying, and/or supplementing the identification of, the patient model at step 52. Examples of such additional resources may include, but are not limited to, published literature, published results of clinical trials, experience gained from determining patient models for other patients, and the like. One or more computer-accessible databases may be made available that contain patient model structures, and that may further contain relevant links to published literature. Example clinical trials from which patient model structure may be determined include, but should not be limited to, tracer studies, and the like. In one exemplary embodiment, patient model structure and parameters may be determined using conventional software, 3$^{rd}$ party software, such as MATLAB ®, SAAM II®, NonMem ®, or some other commercially available software for parameter identification and which may additionally provide the underlying structure of the patient model, provide the model parameters and their initial values, provide the so-called "a priors" if a Bayesian approach is followed, set up a cost function, select an appropriate solver and solve for parameter estimates.

From step 52, the process 50 advances to step 54 where the ability of the patient model identified at step 52 to simulate the patient's physiological response to at least one patient event or condition is validated. Illustratively, this step is implemented via one or more computer-based simulations which are also referred to as specialized test scenarios. Illustratively, step 54 may accomplish one or more of the following: 1) validate the model over one or more specified operating ranges, 2) understand the operating space and limitations of the model, 3) provide estimate(s) of error(s) underlying model assumptions, and 4) utilize multiple models and scheduling, e.g., gain scheduling, to make changes to the model and/or to accurately described a patient's dynamic behavior over the anticipated gluco-regulatory state space. In any case, once the patient model(s) is identified and model parameters determined/adjusted, step 54 may typically include using the patient model to simulate problems, analyze different operating scenarios and examine its dynamic characteristics.

Referring now to FIGS. 4-6, an example patient model 90 is constructed using a number of compartmental model blocks 85 that simulate the distribution of drugs through various organs and areas of the human body. One can construct such models based on the following references: "Applied Biopharmaceutics & Pharmacokinetics," Leon Shargel and Andrew B.C. Yu; "Pharmacokinetics, Principles and Applications," Mehdi Boroujerdi; "A physiologic model of glucose metabolism in man and its use to design and assess improved insulin therapies for diabetes," John Thomas Sorensen, PhD, MIT 1985; "Textbook of Work Physiology, Physiological bases of Exercise", Per-Olof Astrand, Kaare Rodahl, Hans A. Dahl and Sigmund B. Stromme; "Artificial Endocrine Pancreas," Motoaki Shichiri; "The minimal model approach and determinants of glucose tolerance", Richard Bergman and Jennifer C. Lovejoy, Penington Center Nutrition Series, Vol. 7; and "Feedback control in Anaesthesia," Marco Paolo Derighetti, PhD Swiss Federal Institute of Technology, Zurich. The patient model 90 is, in keeping with the example that is common throughout this document, configured to simulate the effect of a meal, e.g., carbohydrates, on blood glucose levels. In one embodiment, the model 90 is used to define a patient interface that the patient may use to input information which characterizes a patient event in the form of patient-specific meal input information of a meal the patient is likely to consume, which is mapped to one or more meal boluses of insulin or other glucose-lowering drug(s). The term "likely to consume" means, in one illustrative embodiment, that the solution set covers about 70% to about 90% of the various meal type, speed, and size combinations. The remaining percentage of meal type, speed, and size possibilities may be handled as exceptional cases. Such exceptional cases will typically be addressed by the patient with an appropriate level of caution and managed by additional monitoring having an acceptable marker of performance in achieving euglycemic control. One such commonly accepted marker, for example, is HbA1C, having a typical target value of 6% or lower, although this numerical example should not be considered to be limiting in any way.

In this example, the generic mathematical description for the patient model is according to equations (1) and (2), which are as follows:

$$\dot{Z}(t) = f_z(Z(t), U(t), t, \theta(t))$$

(1)

and

$$Y(t) = f_y(Z(t), U(t), t, \theta(t))$$

(2),

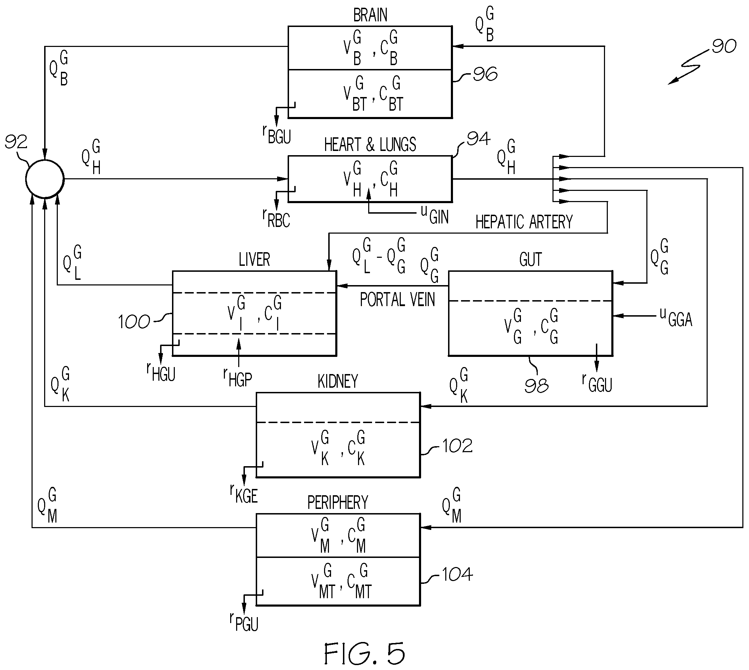

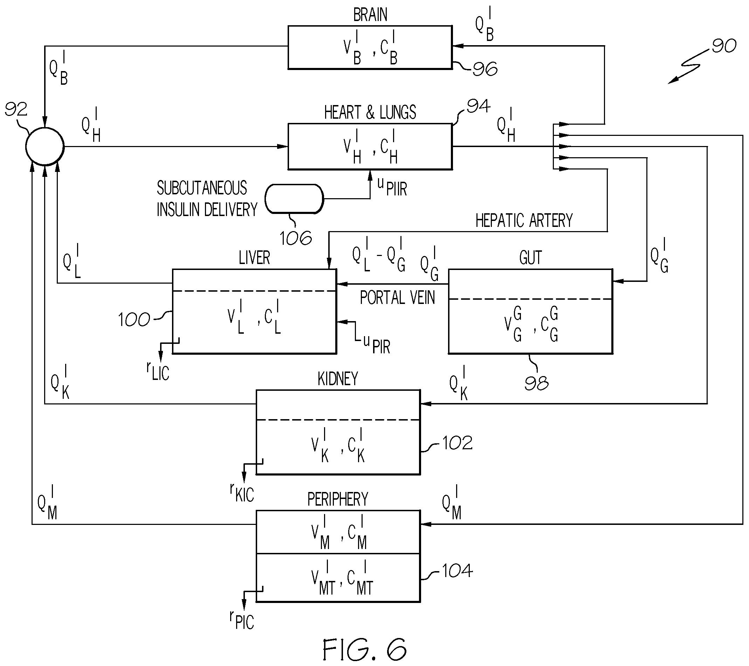

where upper case letters indicate vector quantities and lower case letters indicate scalar quantities. The functions $f_z$ and fy represent the system structure and thereby emulate the characteristic behavior of the diabetic patient. In the model 90, the state vector $Z(t)$ represents state in various compartments of the body such as, for example, heart and lungs 94, brain 96, gut 98, liver 100, kidney 102, and periphery 104 as illustrated in FIGS. 5 and 6, and generally, are either physiologically or non-physiologically based. Depending on the problem requirement, the states represent glucose, insulin, glucagon, FFA, lactate, GLUT, metabolites infused via different modes such as subcutaneous, intravenous, and/or gut. The states included or excluded will generally depend upon the problem to be solved, relevance to the problem, impact of the state on the problem and so forth. Generally, the states change as a function of the input(s), $U(t)$, which represent exogenous and endogenous effects such as endogenous insulin production by pancreas, endogenous glucose production by liver, exogenous glucose infused via intravenous, subcutaneous insulin injection and so on. The state also changes if the state is not in steady state. The parameter $\theta(t)$ is in general a time varying parameter. The output vector $Y(t)$ normally represents physically measurable quantities. However, output equations in general represent quantities of interest. Model representations can become very complex and selection of a final model weigh in complexity, identifiability of parameters, relevant level of detail, and the problem requirement.

Although the structure of the patient model may be obtained in numerous different ways, some of which are described hereinabove, the compartmental block 85 of FIG. 4 is used to simulate the distribution of metabolite in various organs and areas of the body. Referring to the compartmental block 85 of FIG. 4, the concentration of a metabolite may be viewed as an arterial inflow and venous outflow according to the equations:

$$V_B{}^*dC_{BO}/dt = Q_B(C_{Bi} - C_{BO}) + PA(C_I - C_{BO}) + r_{SOURCE1} - r_{SINK1} \qquad (3)$$

and

$$V_I{}^*dC_I/dt = PA(C_{BO} - C_I) - r_{SINK2} + r_{SOURCE2} \qquad (4),$$

where $V_B$ = capillary blood volume, $V_I$ = interstitial fluid volume, $Q_B$ volumetric blood flow rate, PA = permeability-area product, $C_{Bi}$ = arterial blood solute concentration, $C_{BO}$ = capillary blood (and venous) solute concentration, $C_1$ = interstitial fluid solute concentration, $r_{Sink1}$, $r_{Sink2}$ = rate of red blood cell uptake of metabolite, and $r_{Source1}$, $r_{Source2}$ = rate of tissue cellular production of metabolite through the cell membrane. The terms $V_B{}^*dC_{BO}/dt$ and $V_I{}^*dC_I/dt$ represent accumulation, the terms $Q_B(C_{Bi} - C_{BO})$ and $PA(C_{BO} - C_I)$ represent convection, the term $PA(C_I - C_{BO})$ represents diffusion and the terms $r_{Sink1}$, $r_{Sink2}$, $r_{Source1}$ and $r_{Source2}$ represent metabolic sources and sinks.

Using the basic compartmental block structure illustrated in FIG. 4, the model 90 is constructed, as shown in FIG. 5 and 6, so as to represent the concentration of glucose and insulin in various compartments of the body. The states of the various blocks represent the dynamic behavior of the model at any given time, t, relative to various inputs. FIG. 5 represents a schematic for the gluco-regulatory model 90 that defines glucose concentration in the various organs and other areas of the body. FIG. 6 represents a schematic for the gluco-regulatory model 90 that defines insulin kinetics in the various organs and other areas of the body. In each case, a summation node 92 sums the effects of gut 98, liver 100, kidney 102, periphery 104 and brain 96 compartmental blocks, and supplies this summed vector value to a heart and lungs compartmental block 94. Various scalar quantities carried by the output vector of the heart and lungs compartmental block 94 are distributed as inputs to corresponding ones of the gut 98, liver 100, kidney 102, periphery 104 and brain 96 blocks as shown. In the insulin kinetics schematic of FIG. 6, subcutaneous insulin delivery represents a vector input to the heart and lungs block 96.

The output vector, $Y(t)$, of equation (2) above typically comprises physiological quantities such as plasma glucose concentration and plasma insulin concentration which model physically measurable quantities such as glucose concentration using a subcutaneous glucose measuring device. The output vector, Y(t), is typically a function of the state vector, Z. The input vector represents the external and internal influences such as administered insulin, ingested meals, exercise, illness, etc. The overall model 90 represents the particular patient with diabetes.

Referring again to FIG. 2, the steps 52 and 54 of the process 50 represent the development of the patient model that is configured to simulate the patient's physiological response to at least one patient event or condition. Following step 54, the process 50 advances to step 56 where patient-specific information is collected over a period of time that relates to actual occurrences of the at least one patient event or condition. Generally, step 56 is carried out by the patient over an extended time period, e.g., a week to several months, using a manual log book, questionnaire, electronic information recording device, or the like. Using the example that is common throughout this document, a patient may carry out step 56 in a diabetes control system in which information that characterizes patient intake of meals, e.g., carbohydrates, is mapped by the graphical interface to one or more corresponding insulin bolus(es). The patient, in this embodiment, will typically be directed by the patient's physician or other health care provider, or via preprogrammed instructions on an electronic device such as the device 12 of FIG. 1, to log specific meal and insulin related information over a designated time period, wherein the protocol for collection is specified. Generally, the patient will record or log meal times, meal types, meal quantities (in carbohydrate amount), insulin administered before and after meals, blood glucose measurements taken before and after meal consumption, and the like. A more detailed list of such information, including optional and/or alternate information, is described in commonly assigned and co-pending U.S. Patent Application US 2007/0179434 A1, entitled "SYSTEM AND METHOD FOR DETERMINING DRUG ADMINISTRATION INFORMATION".

Following step 56, the process 50 advances to step 58 where a suitable graphic interface is selected (by the user, HCP, or via an algorithm) that will map patient input that characterizes the at least one patient event or condition validated at step 54 to therapy information. In one embodiment, the therapy information is drug therapy information corresponding to one or more drugs to be administered to the patient. In other embodiments, the therapy information may be suggested carbohydrate intake information corresponding to a recommendation to ingest carbohydrates, a recommendation to undertake exercise, a recommendation to consult a physician, and other like therapies. Generally, the graphical interface will take the form of a two-dimensional space defining a characteristic of one patient event or condition along one axis and another characteristic of the patient event or condition along another axis. Again using the example that is common throughout this document, a physician or other health care provider may carry out step 58 in a diabetes control system in which information that characterizes patient intake of meals, e.g., carbohydrates, is mapped by the graphical interface to one or more corresponding insulin bolus(es). Generally, the concentration of glucose in a person changes as a result of one or more external influences such as meals and exercise, and also changes resulting from various physiological mechanisms such as stress, illness, menstrual cycle and the like. In a person with diabetes, such changes can necessitate monitoring the person's blood glucose level and administering insulin or other blood glucose altering drug, e.g., glucose lowering or raising drug, as needed to maintain the person's blood glucose within desired ranges. The system 10 may thus be configured in this example to determine, based on some amount of patient-specific information, an appropriate amount, type and/or timing of insulin or other blood glucose altering drug to administer in order to maintain normal blood glucose levels without causing hypoglycemia or hyperglycemia.

When a person ingests food in the form of a meal or snack, the person's body reacts by absorbing glucose from the meal or snack over time. Any ingesting of food may be referred to hereinafter as a "meal," and the term "meal" therefore encompasses traditional meals, e.g., breakfast, lunch and dinner, as well as intermediate snacks, drinks, etc. The general shape of a gut glucose absorption profile for any person rises following ingestion of the meal, peaks at some measurable time following the meal, and then decreases thereafter. The speed i.e., the rate from beginning to completion, of any one gut glucose absorption profile typically varies for a person by meal composition (e.g. fat, protein, fiber, carbohydrate type, etc.), by meal type (e.g., breakfast, lunch, dinner or snack) or time and/or according to one or more other factors, and may also vary from day-to-day under otherwise identical meal circumstances. Generally, feed forward information relating to such meal intake information supplied by the patient to the system 10 should contain, either explicitly or implicitly, an estimate of the carbohydrate content of the meal or snack, corresponding to the amount of carbohydrates that the patient is about to ingest, is ingesting, or has recently ingested, as well as an estimate of the speed of overall glucose absorption from the meal by the patient.

The estimate of the size or amount of an event or condition that the patient is about to experience, is experiencing, or has recently experienced, may be provided by the patient in any of various forms. For example, but not limited to, the estimate of the amount of carbohydrates that the patient is about to ingest, is ingesting, or has recently ingested, may be provided by the patient as a direct estimate of carbohydrate weight (e.g., in units of grams or other convenient weight measure), an amount of carbohydrates relative to a reference amount (e.g., dimensionless), an estimate of meal or snack size (e.g., dimensionless), and an estimate of meal or snack size relative to a reference meal or snack size (e.g., dimensionless). Other forms of providing for patient input of carbohydrate content of a meal or snack will occur to those skilled in the art, and any such other forms are contemplated by this disclosure.

The estimate of the speed of the event or condition that the patient is about to experience, is experiencing, or has recently experienced, likewise may be provided by the patient in any of various forms. For example, but not limited to, for a specified value of the expected speed of overall glucose absorption of a meal, the glucose absorption profile captures the speed of the meal taken by the patient. As another example, the speed of overall glucose absorption from the meal by the patient also includes a time duration between ingesting of the meal by a person and the peak glucose absorption of the meal by that person, which captures the duration of the meal taken by the patient. The speed of overall glucose absorption may thus be expressed in the form of meal speed or duration. Examples of the expected speed of overall glucose absorption parameter in this case may include, but are not limited to, a compound parameter corresponding to an estimate of the meal speed or duration (e.g., units of time), a compound parameter corresponding to meal speed or duration relative to a reference meal speed or duration (e.g., dimensionless), or the like.

As another example of providing the estimate of the expected speed of overall glucose absorption parameter, the shape and duration of the glucose absorption profile may be mapped to the composition of the meal. Examples of the expected speed of overall glucose absorption parameter in this case may include, but are not limited to, an estimate of fat amount, protein amount and carbohydrate amount (e.g., in units of grams) in conjunction with a carbohydrate content estimate in the form of meal size or relative meal size, an estimate of fat amount, protein amount and carbohydrate amount relative to reference fat, protein and carbohydrate amounts in conjunction with a carbohydrate content estimate in the form of meal size or relative meal size, and an estimate of a total glycemic index of the meal or snack (e.g., dimensionless), wherein the term "total glycemic index" is defined for purposes of this document as a parameter that ranks meals and snacks by the speed at which the meals or snacks cause the person's blood sugar to rise. Thus, for example, a meal or snack having a low glycemic index produces a gradual rise in blood sugar whereas a meal or snack having a high glycemic index produces a fast rise in blood sugar. One exemplary measure of total glycemic index may be, but should not be limited to, the ratio of carbohydrates absorbed from the meal and a reference value, e.g., derived from pure sugar or white bread, over a specified time period, e.g., 2 hours. Other forms of providing for user input of the expected overall speed of glucose absorption from the meal by the patient, and/or for providing for user input of the expected shape and duration of the glucose absorption profile generally will occur to those skilled in the art, and any such other forms are contemplated by this disclosure.

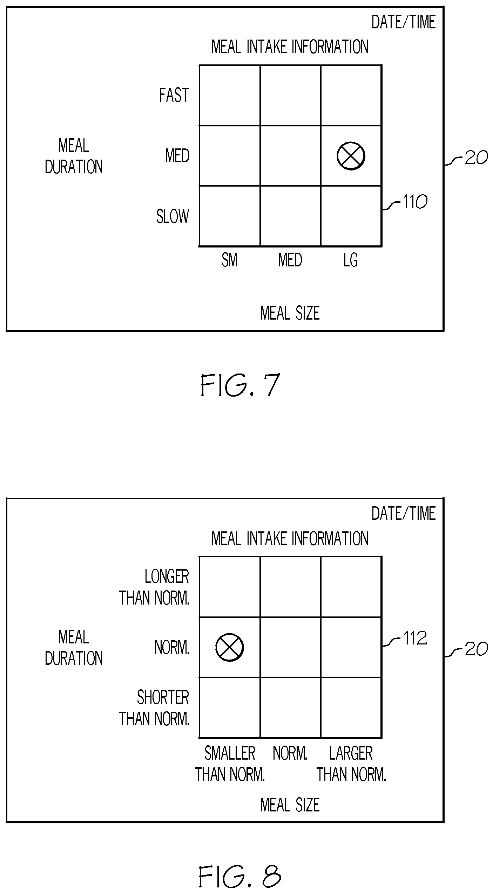

The graphical interface in this example illustratively has a first parameter component and a second parameter component. In one embodiment directed at patient input of meal related information, the first parameter component of the patient input of the meal related information illustratively corresponds to a carbohydrate amount or content of the meal that the patient is about to ingest, is ingesting, or has recently ingested, and the second parameter component illustratively corresponds to an expected speed of overall glucose absorption from the meal by the patient. Referring to FIG. 7, one exemplary embodiment of such a graphical interface 110 selectable to provide patient input of meal intake information is shown. In the illustrated embodiment, the graphical interface 110 is a grid-type user interface having one grid axis defined by carbohydrate content in the form of meal size and another grid axis defined by expected speed of overall glucose absorption from the meal by the patient in the form of meal duration. The meal size grid axis defines three different meal size or amount values in the form of "small", "medium" and "large" indicators, and the meal duration grid axis likewise defines three different meal duration values in the form of "slow", "medium" and "fast" indicators. The grid-type graphical user interface 110 provides for a single user selection of carbohydrate content and expected speed of overall glucose absorption information relating to the meal that the patient is about to ingest, is ingesting, or has recently ingested. As used herein, the phrase "single user selection" is defined as a single selection made by a user. It will be understood that the systems and methods described herein are not limited to a single user, and that rather the systems and methods described in this document may be implemented in a single or multiple user platform. In any case, the user has selected, in the illustrated example, a meal related input indicating that the meal that the patient is about to ingest, is ingesting, or has recently ingested is a large meal that will be, or that has been, ingested over a medium meal duration. In general, the terms "large," "medium," and "small" in this context are intended to encompass any conventional measure of meal size including for example, but not limited to, meal quantities or amounts using any specified units of weight, volume, etc.

Referring to FIG. 8, another exemplary embodiment of a graphical interface 112 selectable to provide user input of meal intake information is shown. In the illustrated embodiment, the graphical interface 112 is a grid-type interface having one grid axis defined by carbohydrate content in the form of meal size relative to a reference meal size and another grid axis defined by expected speed of overall glucose absorption from the meal by the patient in the form of meal duration relative to a reference meal duration. The meal size grid axis defines three different meal size values in the form of "smaller than normal", "normal" and "larger than normal" indicators, and the meal duration grid axis likewise defines three different meal duration values in the form of "shorter than normal", "normal" and "longer than normal" indicators. The grid-type graphical interface 112 provides for a single user selection of carbohydrate content and expected speed of overall glucose absorption information relating to the meal that the patient is about to ingest, is ingesting, or has recently ingested. In the illustrated example, the user has selected a meal related input indicating that the meal that the patient is about to ingest, is ingesting, or has recently ingested is a smaller than normal meal and that the meal duration is about the same as a normal meal duration. In general, the terms "larger" and "smaller" in this context are intended to encompass any conventional measure of meal size relative to a specified "normal" meal size including for example, but not limited to, meal quantities or amounts using any specified units of weight, volume, etc.

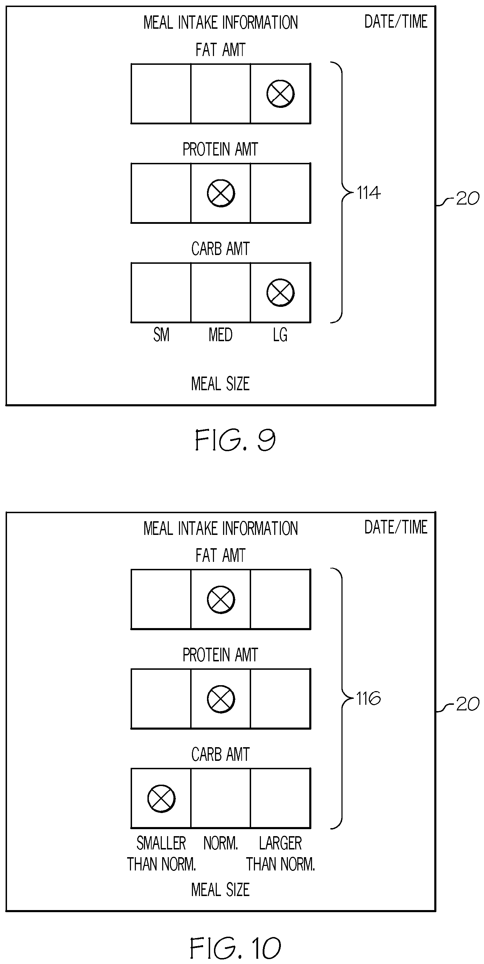

Referring to FIG. 9, yet another exemplary embodiment of a graphical interface 114 selectable to provide patient input of meal intake information is shown. In the illustrated embodiment, the graphical interface 114 is a grid-type user interface having one grid axis defined by carbohydrate content in the form of meal size and another grid axis defined by expected speed of overall glucose absorption in the form of fat amount, protein amount and carbohydrate amount of the meal. The graphical interface 114 thus requires three separate selections to be input by the patient, as compared with the single input associated with the embodiments illustrated and described with respect to FIGS. 7 and 8. The fat amount, protein amount and carbohydrate amount is mapped, as described briefly hereinabove, to an expected speed of overall glucose absorption from the meal by the patient. The meal size grid axis defines three different meal size values in the form of "small", "medium" and "large" indicators. The grid-type graphical interface 114 provides for the user selection of carbohydrate content and expected speed overall glucose absorption information relating to the meal that the patient is about to ingest, is ingesting, or has recently ingested. In the illustrated example, the patient has selected a meal related input indicating that the meal that the patient is about to ingest, is ingesting, or has recently ingested has a large amount of fat, a medium amount of protein and a large amount of carbohydrates. In general, the terms "large," "medium," and "small" in this context are intended to encompass any conventional measure of meal size including for example, but not limited to, meal quantities or amounts using any specified units of weight, volume, etc.

Generally, any desired functional relationship may be used to map the three meal composition amounts to corresponding meal speed or meal duration values. One exemplary functional relationship may be, but should not be limited to, assigning equal weights to the three meal composition components, computing percentages of the three user-specified meal composition values, assigning equally spaced thresholds to the two interfaces between the three meal size values, e.g., 33% and 66%, and then comparing the percentages of the three meal composition values to the threshold percentage values to determine meal speed. Using the example illustrated in FIG. 9, the small, medium and large components are assigned values of 1, 2 and 3 respectively. The percentage of fat is thus 3/8 or 37.5%, the percentage of protein is 2/8 or 25%, and the percentage of carbohydrates is 3/8 or 37.5%. The percentages of fat and carbohydrates are thus both medium, and the percentage of protein is small, resulting in a composite meal speed of medium to medium-slow.

Referring to FIG. 10, still another exemplary embodiment of a graphical interface 116 selectable to provide patient input of meal intake information is shown. In the illustrated embodiment, the graphical interface 116 is a grid-type user interface having one grid axis defined by carbohydrate content in the form of meal size relative to a reference meal size and another grid axis defined by expected speed of overall glucose absorption from the meal by the patient in the form of fat amount, protein amount and carbohydrate amount. As with the graphical interface 114, the graphical interface 116 thus requires three separate selections to be input by the user, as compared with the single input associated with the embodiments illustrated and described with respect to FIGS. 7 and 8. The user-specified fat, protein and carbohydrate amounts is mapped to corresponding meal speed or meal duration values using any desired functional relationship therebetween as just described. The meal size grid axis defines three different meal size values in the form of "smaller than normal", "normal" and "larger than normal" indicators. The grid-type graphical interface 116 provides for user selection of carbohydrate content and expected speed of glucose absorption information relating to the meal that the patient is about to ingest, is ingesting, or has recently ingested. In the illustrated example, the user has selected a meal related input indicating that the meal that the patient is about to ingest, is ingesting, or has recently ingested has a normal fat amount, a normal protein amount and a smaller than normal carbohydrate amount. In general, the terms "larger" and "smaller" in this context are intended to encompass any conventional measure of meal size relative to a specified "normal" meal size including for example, but not limited to, meal quantities or amounts using any specified units of weight, volume, etc.

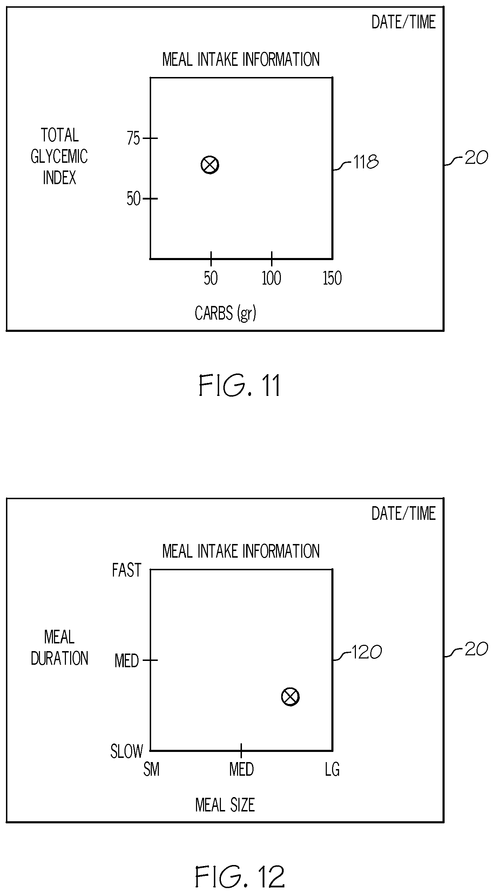

Referring to FIG. 11, a further exemplary embodiment of a graphical interface 118 selectable to provide user input of meal intake information is shown. In the illustrated embodiment, the graphical interface 118 defines a continuous function of the carbohydrate content, provided in the form of carbohydrate content by weight (in grams or other convenient weight units) and expected speed of overall glucose absorption from the meal by the patient provided in the form of a total glycemic index (dimensionless). Alternatively, the graphical interface 118 could define a numeric display that is a discrete function of the carbohydrate content, provided in the form of carbohydrate content and expected speed of glucose absorption provided in the form of a total glycemic index. In either case, the carbohydrate content and/or total glycemic index parameters may alternatively be expressed in the graphical user interface 60 in the form of "large," "medium," and "small," as these terms are described hereinabove, or in the form of "larger than normal," "normal," and "smaller than normal," as these terms are described hereinabove. Any number of dotted, dashed, solid or other types of grid lines may alternatively or additionally be superimposed onto the graphical user interface 58 to facilitate discrimination between carbohydrate content and total glycemic index values on the interface 118. In any case, the graphical interface 118 provides for a single user selection of carbohydrate content and expected speed of glucose absorption information relating to the meal that the patient is about to ingest, is ingesting, or has recently ingested. In the illustrated example, the user has selected a meal related input indicating that the meal that the patient is about to ingest, is ingesting, or has recently ingested has a carbohydrate weight of approximately 50 grams and a total glycemic index value of approximately 62.

Referring to FIG. 12, still another exemplary embodiment of a graphical interface 120 selectable to provide user input of meal intake information is shown. In the illustrated embodiment, the graphical interface 120 defines a continuous function of the carbohydrate content, provided in the form of meal size, and expected speed of overall glucose absorption from the meal by the patient provided in the form of meal duration. The meal size axis defines three different meal size values in the form of "small", "medium" and "large" indicators, and the meal duration axis likewise defines three different meal duration values in the form of "slow", "medium" and "fast" indicators. The continuous-type graphical interface 120 provides for a single user selection of carbohydrate content and expected speed of overall glucose absorption information relating to the meal that the patient is about to ingest, is ingesting, or has recently ingested. Any number of dotted, dashed, solid or other types of grid lines may alternatively or additionally be superimposed onto the graphical interface 120 to facilitate discrimination between meal size and meal duration values on the interface 120. In the illustrated example, the user has selected a meal related input indicating that the meal that the patient is about to ingest, is ingesting, or has recently ingested is between medium and large size and ingested at a meal duration between slow and medium.

Further details and examples of graphical interfaces are illustrated and/or described in commonly owned and co-pending U.S. Patent Application US 2007/0179434 A1.

Referring again to FIG. 2, the process 50 advances from step 58 to step 60 where the solution space of the graphical interface selected at step 58 is defined based on the patient model resulting from steps 52 and 54, and also based on the patient-specific information collected pursuant to step 56. The solution space defines the relationship between inputs and their associated acceptable limits to regulate a patient's physiological response to a desired target response. Again using the example that is common throughout this document, a physician or other health care provider may carry out step 60 in a diabetes control system in which information that characterizes patient intake of meals, e.g., carbohydrates, is mapped by the graphical interface to one or more corresponding insulin bolus(es). In this specific example, the graphical interface is a grid-type interface defined by the two input parameters meal amount and meal speed, e.g., the graphical interface 110 of FIG. 7. It will be understood, however, that any of the graphical interface embodiments illustrated and described herein or other similar graphical interfaces could alternatively be used. In one illustrative embodiment, the solution space of the graphical interface 110 will generally be determined in a manner that not only defines the "grid" locations, i.e., the divisions between the different meal size and meal speed (duration) inputs, but that also defines the outer boundary of the graphical interface 110. In any case, it is desirable to define the solution space of the graphical interface 110 such that it provides for the regulation of the patient's glucose relative to a target glucose level for a given meal disturbance.

As illustrated in FIG. 7, the meal space is divided into contiguous rectangular sub-spaces. These sub-spaces represent a range of meal characterizations that can each be repeatedly accommodated by a common insulin therapy. By defining the solution space of the graphical interface, a subspace of meal information is identified within which fixed meal compensation is acceptable. Further for illustrative purposes, considering the glycemic control problem wherein the physiological variable glucose concentration g(t) is regulated, one of the output elements in the vector Y(t) is g(t). Illustratively, then, the solution space of the graphical interface may be determined by solving a cost function that is set up as a constrained minimization problem. Such a cost function may use, for example, the following information to solve the constrained minimization problem: 1) gluco-regulatory model response due to meal excitation, 2) meal excitation input parameters of meal size (amount), $\eta^A$ and meal speed (duration), $\eta^S$, 3) target glucose value, $g_{target}$, 4) the output vector, Y(t), the state vector, Z(t), and the input vector U(t) (from equations (1) and (2) above), 5) weighting parameters, and 6) constraints on glucose values (e.g. maximum and minimum glucose values) and insulin administration amount.

One example cost function is shown by equation (5) which is as follows:

$$ J = \int_{t1}^{t2} h(Z(t),U(t),t)dt + h_0(g_{target}, Z(t_0)) + h_f(g_{target}, Z(t_f)) \qquad (5), $$

where h is a scalar function, and $h_0$ and $h_f$ are initial and final cost parameters. The cost function is solved by minimizing J, and is further required to satisfy the following constraints: 1) $g \geq g_{min}$, 2) $g \leq g_{max}$, 3) $dg/dt \leq \dot{g}_{max}$ and 4) $dg/dt \geq \dot{g}_{min}$, where "g" is the glucose concentration, $g_{min}$ is the lower glucose limit for euglycemia, $g_{max}$ is the upper glucose limit for euglycemia, $\dot{g}_{max}$ is the maximum rate of increase of glucose concentration and $\dot{g}_{min}$ is the minimum rate of decrease of glucose concentration. Furthermore, the above described constraints can be a function of time and state vector. The meal space is defined by $\eta^A$ and $\eta^S$ subject to the constraints: 1) $n^A \geq \eta_1^A$, 2) $\eta^A \leq \eta_2^A$, 3) $\eta^S \geq \eta_1^S$ and 4) $\eta^S \leq \eta_2^S$, where $\eta^A \; \varepsilon \; [\eta_1^A, \eta_2^A]$ and $\eta^S \; \varepsilon \; [\eta_1^S, \eta_2^S]$ are parameter ranges over which the meal size (amount) and meal speed (duration) are expected to vary respectively. It should be noted that for illustrative purposes the gut glucose absorption characteristics is assumed to be described by $\eta^A$ and $\eta^S$.

The grid size of the graphical interface 110 in FIG. 7 is determined using a predictive algorithm that illustratively uses predictions of the patient model to future values or states to determine an optimum or "best" control action until the next computation iteration. A non linear model predictive controller is one possible candidate for solving the above cost function constraints. The non linear model controller approach requires an explicit use of a model to predict future behavior. The method utilizes past input information and determines a control action based on cost (objective function). The method by choice can consider future control action also to provide an overall best solution. This method is flexible and generalizes to future extensions such as considering an additional input or disturbance without any special treatment. Several references covering this topic are: "Stabilizing state feedback design via the moving horizon method", Kwon, Bruckstein and Kailath, Intl. Journal of Control, 37, 1983, pp. 631-643; "Model predictive control: theory and practice", Garcia, Prett and Morari, Automatica, 25, 1989, pp. 335-348; and "Nonlinear model predictive control of glucose concentration in subjects with type 1 diabetes", Roman Hovorka, Valentina Canonico, Ludovic J Chassin, Ulrich Haueter, Massimo Massi-Benedetti, Marco Orsini Federici, Thomas R Pieber, Helga C Schaller, Lukas Schaupp, Thomas Vering and Malgorzata E Wilinska, 2004 Physiol. Meas. 25 905-920. A predictive algorithm with Bayesian parameter estimation, as described by Romon Hovorka, et. al., would complement the model predictive approach with on-line, model adaptive capability by also performing parameter identification. Without loss in generality, non linear predictive algorithms may be implemented also as an open loop implementation as suggested herein, wherein meal compensation bolus values are determined for a given meal type ($\eta^A$, $\eta^S$) for a given set of initial conditions, parameter values and weights. Thus a single point solution is obtained when the predictive controller is set up and solved. Furthermore, the solution is not specific to using the model predictive approach, but illustrates the intent of the solution. The intent is to determine a single optimal therapy from a potential of many admissible solutions.

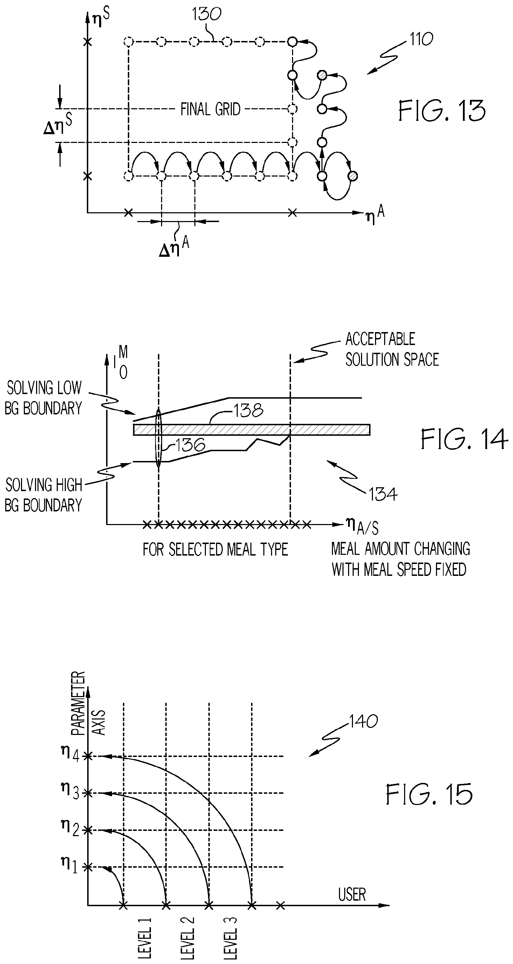

The total meal space is defined in this example by the parameters $\eta^A$, which corresponds to the meal size or amount, and $\eta^S$, which corresponds to the meal speed or duration. Ranges for the parameters $\eta^A$ and $\eta^S$ are defined by the actual meal-related information that the patient collects pursuant to step 56 of the process 50. To define the therapy solution over the meal space in terms of the graphical interface, the $\eta^A$ and $\eta^S$ ranges are used to specify the outer periphery or boundaries of the graphical interface. This is illustrated graphically by example in FIG. 13, which illustrates the formation of the outer periphery 130 of the graphical interface 110 based on the ranges of $\eta^A$ and $\eta^S$. In the illustrated example, the ranges of the parameters $\eta^A$ and $\eta^S$ are divided into small step sizes $\Delta\eta^A$ and $\Delta\eta^S$ respectively. The division of step sizes depends on solution sensitivity to step size. Small step sizes may be needed, for example, for patients who are insulin sensitive. Normally dividing the overall range in 10 to 15 equally spaced segments is typically sufficient. However more or fewer may be needed depending on problem needs. The predictive algorithm is then solved to cover the meal space by "walking" around the perimeter 130 using the small, discrete steps $\Delta\eta^A$ and $\Delta\eta^S$ as illustrated in FIG. 13. Another possibility for mapping the meal space is to divide the operational range into a mesh and to then solve the problem as described hereinafter at each of the intersecting grid points. Yet another possibility may include, for example, defining one or more simplified analytical functions that encompass the therapy solution, and then using this equivalent function as a solution space for determining a therapy solution for various grids (e.g., see FIGS. 7 through 12). More specifically, the constrained minimization problem represented by equation (5) above is solved at each step two times. To solve for meal compensation bolus amount, the predictive algorithm uses the following information: 1) $\eta^A$ and $\eta^S$, 2) the cost function (equation (5)), 3) all states of the patient model are set to steady state, and insulin administration is set to an appropriate basal rate, and 4) assuming the meal or snack is consumed at time $t^M_0$, meal-related boluses are defined as ...., $I^M_{-k1}$, $I^M_{k0}$, $I^M_{k1}$, $I^M_{k2}$, ... wherein, $I^M_{ki}$, represents a meal bolus at various times (ki minutes) relative to the meal time (k0=0 minutes). The term with -k1, -k2 and so on represents the time, e.g., in minutes, with respect to ingestion of meal at time 0. Typically, the solution may define a series of finite meal insulin boluses. Furthermore, the boluses may be defined as a number of fractions of a single meal insulin bolus, e.g., consider 4 boluses $I^M_{-15}$=10% of I, $I^M_0$ =70% of I, $I^M_{15}$ =10% of I, and $I^M_{60}$ =10% of I. However, for illustrative purposes the simplest case is considered herein, e.g., a single meal bolus is administered at the time the meal is consumed, i.e., only $I^M_0$.

As illustrated in FIG. 13, the graphical interface periphery 130 is spanned by covering the various meal characteristics. For each selected meal characteristic, the predictive algorithm is solved to determine the meal-related insulin compensation. The predictive algorithm is set up as a constrained minimization problem in which, for a given set of weighting functions, control-related constraints, system equations with given past, current and future input excitation (e.g. meal ingestion, glucose measurements, insulin infused), constraints on the output (e.g. insulin infusion is non-negative, glucose concentration has to achieve target glucose or stay within upper and lower boundaries, and so forth), and constraints on the input (e.g. maximum insulin bolus), equation (5) may be written as

$$J = \int_{t1}^{t2} [(1/2)Z^T W_z Z + (1/2)U^T W_U U]dt + (1/2)Z^T_0 W_0 Z_0 + (1/2)Z^T_f W_f Z_f \qquad (6).$$

Regarding the constraints on the input, the meal-related boluses are limited to a single meal bolus, $I^M_0$, so that the input vector, U, corresponding to meal insulin is given as U = [0 $I^M_0$ 0 0 ... 0]. Details of various model predictive control (MPC) embodiments are provided in the references listed above.

At each meal characterizing point illustrated in FIG. 13, the permissible solution space is captured by solving two separate minimization problems: 1) minimum of maximum insulin administration for violating a lower glucose limit, and 2) maximum of minimum insulin administration for violating an upper glucose limit. For the first minimization problem, the cost function is set up with small weights for the control function, which results in small penalties for control action. This forces a solution set that determines the control action with liberal use of insulin in a manner that does not violate the lower glucose limit, $(I^M_0)_{maximum}$. From all the admissible solution set the cost function determines the maximum insulin administration without violating the lower glucose constraint. For the second minimization problem, the cost function is set up with large weights for the control function, which results in large penalties for control action. This forces a solution set that determines the control action with minimal insulin use in a manner that does not violate the upper glucose limit, $(I^M_0)_{minimum}$.. The set of solutions are then all the solutions between $((I^M_0)_{minimum}$ and $(I^M_0)_{maximum})$ which will satisfy the constraints and that provide glycemic control to meal intake $\eta^A$ and $\eta^S$ for given set of parameters and conditions.

As the predictive algorithm advances from point to point along the periphery of the 130 of the graphical interface 110 as illustrated in FIG. 13, the solution at each point is recorded. An example of one technique for recording such solutions is illustrated in FIG. 14, which is a plot 134 of meal bolus quantity vs. the total meal space $\eta^{A/S}$, and which illustrates the solution space extremum. It is to be appreciated that FIG. 14, could also be represented as a three dimensional figure in which $\eta^A$ is shown separately from $\eta^S$ as the point should be clear that in FIG. 14, $\eta^{A/S}$ represents two axis overlapped showing the resulting common space of interest. Alternatively, from a computational aspect, the solution is maintained in the memory for further analysis and determination of a grid. For each point along the periphery 130 of the graphical interface 110, a solution is sought by systematically varying one parameter at a time. The step size, $\Delta$, for $\eta^A$ and for $\eta^S$ is typically problem specific. If a solution is found, the solution space is saved and the iterative process proceeds to the next point. If no solution is found, the periphery point is rejected, the iterative process moves back to the previous point, and the iterative process proceeds in a direction normal to the previous direction of travel, as illustrated in FIG. 13. In this manner, the total meal space $\eta^{A/S}$ is systematically spanned, and the solution space for the meal space $\eta^{A/S}$ is determined. For example, if representing the above approach with a 3-D figure, then the solution space is bounded by upper and lower surfaces, wherein the surfaces are formed by connecting the solution points by straight lines.

The division of the meal space into grid-like subspaces, as illustrated in FIG. 11 for example, may be determined via further analysis of the solution space. The objective is to find a simply connected continuous region of the solution space. Referring again to FIG. 14, for example, the elliptical region 136 corresponds to the solution range for a specific point in meal space, and the solid band 138 corresponds to the common solution sub-space covering a range of meal space. In one embodiment, normally the solution space of acceptable therapy is a three-dimensional space where $\eta^A$ and $\eta^S$ are the x and y axis with $I^M_0$ on the vertical z-axis. The grid-like subspaces may be generated by a schema, for example, wherein the band 138 not only covers the meal space continuously, but wherein additional constraints may be added as needed or as desired in view of the desired or required therapy. Those skilled in the art will recognize that more complex or developed strategies may be used to further define the grid-like subspaces, such as one or more strategies that consider the interior points of the space 130.

Further requirements may be imposed on the solution space extremum illustrated in FIG. 14. For example, a minimum margin may be provided from the upper and lower glucose boundaries to manage individual patient sensitivity to parameter variations. Alternatively or additionally, the band 138 may be the feasible solution over the entire selected meal sub-space. Alternatively or additionally still, a straight plane may cut across the entire subspace so that the insulin therapy is satisfied for the subspace of $\eta^A$ and $\eta^S$. Discontinuities may be the natural division lines for a meal sub-space. Alternatively, the subspace may be determined by systematically covering the entire surface by an iterative algorithm that sets up a three-dimensional grid for the solution space that sets up an iterative loop to span the three-dimensional space, that checks conditions at each mesh intersection, and that advances to a next feasible solution when a solution fails. As another example requirement, it may be desirable to require a single solution from the band 138 so that, in general, minimum insulin quantities are recommended or administered. This approach minimizes, or at least reduces, the potential for hypoglycemic conditions. Generally, this example considered only a single input, i.e., meal-related information. Those skilled in the art will recognize that the graphical interface determination process may be extended to alternate or additional inputs, e.g., illness, exercise, stress, etc., using the same concepts just described. However, it is be appreciated that by the above method, that each grid cell is represented by a single point which effectively covers the therapy for the entire grid-cell.

The foregoing method has been described for patient parameters in one particular state, e.g., the solution for the patient is performed when he/she is in a "normal" state as opposed to being in state of stress (also referred as alternate state). This approach can in the same way be applied to various parameter and physiological states. Furthermore the patient model may be considered as a function of time to consider time-based affects such as days, weeks, months, seasonal affects, or the like. As examples, menstrual state for women, seasonal influence on meal composition, amount of meal consumed as function of working and non working days, and so forth.

Once the grid-like graphical interface 110 of this example is sufficiently defined to provide a meal compensation bolus strategy that meets the euglycemic goals, patient inputs must be mapped to the solution. Referring to FIG. 15, for example, a plot 140 of one of the meal space parameters, i.e., either $\eta^A$ or $\eta^S$, vs. discrete user inputs is shown. If the $\eta$ axis represents the continuous range of either $\eta^A$ or $\eta^S$, this parameter can be mapped to any number of discrete user inputs. Thus, the parameters are $\eta_1 \leq n_2 \leq \eta_3 \leq \eta_4$ and $Level_1 \, \varepsilon \, [\eta_1, \eta_2]$ with nominal value $\eta \, \varepsilon \, [\eta_1, \eta_2]$. Generally, this mapping is carried out with respect to each of the meal space parameters. In the example graphical interface 110 illustrated in FIG. 7, the meal size parameter, i.e., $\eta^A$, is mapped to three discrete user inputs, SM, MED and LG, and the meal speed (duration) parameter, i.e., $\eta^S$, is also mapped to three discrete user inputs, SLOW, MED and FAST. Generally, any number of discrete user inputs may be defined, although from a practical standpoint it is desirable to target the number of discrete user inputs to be large enough to distinguish different parameter ranges yet small enough to be manageable by the patient.

It is anticipated that the solution may also be used to monitor the patient state using the patient model. With measured patient-specific data taken over some time period, this allows the patient model to correct for modeling errors. More specifically, the patient model may be used to predict glucose excursion in the future from current and historical information, and can provide for the monitoring of the overall system, for the correction of modeling errors, for determining if the patient model is, or continues to be, appropriate for the patient or whether it requires further/additional development, for the setting and/or prediction of alarm conditions, such as hypo-glycemic and/or hyper-glycemic events, and for predicting when to administer the next meal bolus.

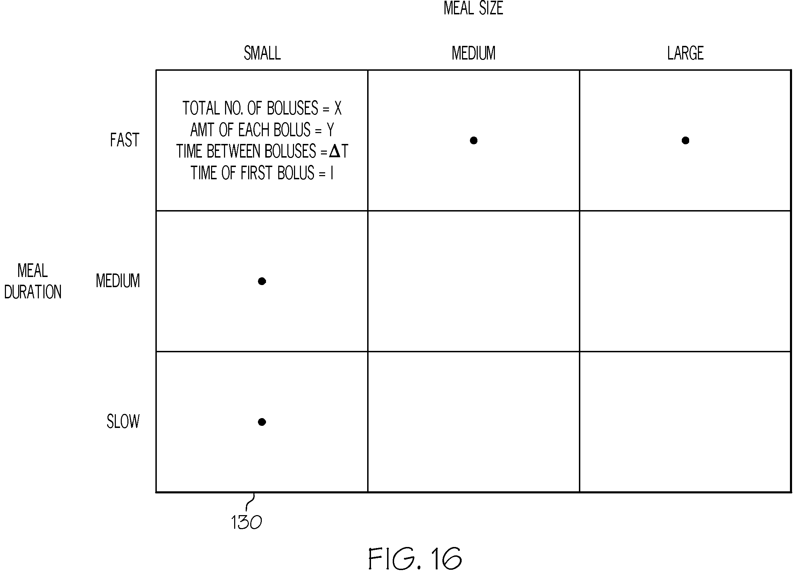

Referring again to FIG. 2, the process 50 advances from step 60 to step 62 where a map is defined that maps patient inputs to the graphical interface to corresponding drug therapy information. Again using the example that is common throughout this document, the memory or data storage unit 16 and/or 34 illustratively has stored therein a map correlating patient-entered meal information to insulin delivery amounts. The map may be provided in any conventional form, examples of which include, but are not limited to, one or more graphs, charts, tables, equations or the like. One exemplary embodiment of such a map 148 is shown in FIG. 16, and is provided in the form of a table mapping carbohydrate content, in the form of meal speed, and expected speed of overall glucose absorption from the meal by the patient, in the form of meal duration, to meal compensation bolus information. In the embodiment illustrated in FIG. 16, the meal compensation bolus information may be or include any one or more of a total number, X, of insulin boluses to be administered to the user, an amount or quantity, Y, of each of the number of insulin boluses to be administered (e.g., in international units), a time, $\Delta T$, between each of the insulin boluses to be administered and a time, I, that a first one of the number of insulin boluses will be administered. Those skilled in the art will recognize that other insulin dosing schemas may be used to define the map correlating the patient-entered meal information to insulin delivery amounts, and any such other insulin dosing schemas are contemplated by this disclosure.

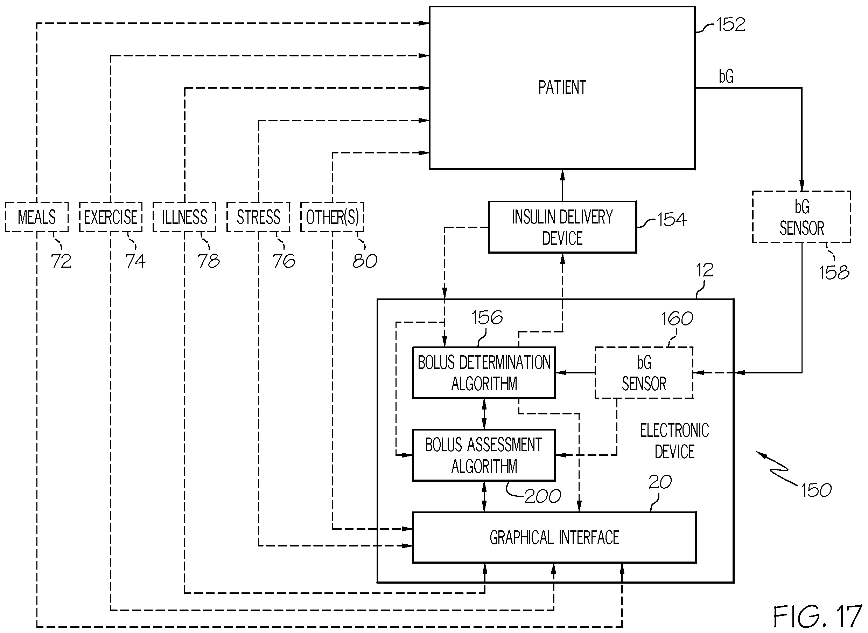

Referring now to FIG. 17, a block diagram of one illustrative embodiment of the electronic device 12 of FIG. 1 implemented in a semi-closed loop drug administration system 150 is shown. The patient, represented by block 152 in the system 150, is simulated by the patient model developed at steps 52 and 54 of the process 50 of FIG. 2. An insulin delivery device 154 is configured to deliver insulin, or one or more other patient glucose raising or lowering drugs, to the patient 152. The insulin delivery device 154 may be a conventional implanted or externally worn infusion pump, and in this embodiment the dashed-line connecting the electronic device 12 to the device 154 may represent a wired or wireless communication path. Alternatively, the insulin delivery device 154 may be a conventional manually actuated device, such as a conventional syringe, insulin pen or the like and in this case the dashed line connecting the electronic device 12 to the device 154 represents only that insulin administration that is recommended by the electronic device 12 is manually administered by the device 154.

The electronic device 12, in the embodiment illustrated in FIG. 17, includes a bolus assessment/adjustment algorithm 200 that receives input from the patient via the graphical interface 20. The patient input to the algorithm 200 is in the form of one or more inputs to the graphical interface 20 as described herein, and the algorithm 200 is generally operable to determine from the patient input an appropriate patient therapy, one example of which may be one or more meal boluses as described herein. It is to be appreciated that the assessment performed by the algorithm 200 is by examining the overrides from the specified therapy and/or corrective actions done in addition to therapy. Thus, the discrepancy between dispensed and commanded is used by the algorithm to assess the effectiveness of the therapy, e.g., to achieve a desired glycemia from bG measurement. In one embodiment, to provide a semi-closed or closed loop assessment/adjustment, devices 154 and/or 158 (or 160) communicate to the algorithm 200 the dispensing information and/or the measurement information.

In the illustrated embodiment, the algorithm 200 may supply its therapy output to the graphical interface 20 for patient review, and/or to a conventional bolus determination algorithm 156 which may incorporate the results of the algorithm 200 therein. Alternatively, the bolus assessment/adjustment algorithm 200 may be incorporated into the bolus determination algorithm 156, in which case the block 200 may be omitted from FIG. 17. In any case, the bolus determination algorithm 156 may supply information to the graphical interface 20 for display, and may provide bolus administration information for the control of the insulin delivery device, as indicated by the dashed-lined arrows extending between the bolus determination algorithm 156 and the graphical interface 20 and insulin delivery device 154 respectively. In some embodiments, the insulin delivery device 154 may provide actual insulin delivery information back to the bolus determination algorithm 156, as indicated by the dashed-line arrow extending between these two blocks.

In addition to the patient input to the graphical user interface 20, the bolus determination algorithm 156 receives blood glucose information from the patient 152 via a blood glucose (bG) sensor. In one embodiment of the system 150, a bG sensor 158 may be external to the electronic device 12, as shown by dashed-line representation in FIG. 17. In this embodiment, the bG sensor 158 may be a conventional implanted or external bG sensor, or a bG sensor providing an on-demand bG. In the case of an external bG sensor, the bG sensor 158 may be any conventional bG sensor, including one that supplies blood glucose information to the device 12 via wired or wireless connection, or one that analyzes a sample of blood and produces a blood glucose reading (e.g., a conventional blood glucose meter) that must be manually entered into the device 12 such as via a keypad or other input device. Alternatively or additionally, a conventional bG sensor 160 may be included on-board the electronic device. In this embodiment, the bG sensor 160 includes a blood analysis facility (e.g., blood glucose meter such as a conventional electrochemical or photometric glucose strip reader) that analyzes a sample of blood, determines a corresponding blood glucose value, and provides the blood glucose value to the bolus determination algorithm. In any case, the conventional bolus determination algorithm 156 is operable to include the blood glucose information in the determination of bolus amounts to be administered.

As illustrated in FIG. 17, the patient 152 is generally subject to various patient-related events and conditions, some of which may include, but should not be limited to, any number of meals 72 (including any consuming of carbohydrates), patient activity such as exercise 74, patient illness 78, patient-related stress 76 or other event(s) or condition(s) 80. As described hereinabove, the graphical user interface 20 may be developed to accommodate any one or more such patient-related events or conditions, one or more patients, and/or multiple graphical user interfaces may be developed to each accommodate a specific one of various patient-related events or conditions. Alternatively, the device may have a mechanical switch, or a mechanical knob or a mechanical dial or selectable software setting which lets the algorithm know of the alternate state so that appropriate therapy parameters can be selected. It is also contemplated that the alternate state may be identifiable by one or more sensor which may then be used in either a manual or an automated fashion to use the alternate state information to select appropriate therapy parameters. In any case, the electronic device 12, in the system 150 illustrated in FIG. 17, is operable to consider any one or more such patient-related events or conditions, along with patient blood glucose information, in determining and one or more appropriate therapies. The device 12 may then control the automatic delivery of drug therapy, and/or recommend any appropriate therapy to the patient, which appropriate therapy may include, but should not be limited to, drug therapy, exercise therapy, a recommendation to consult and/or seek a health care professional, or the like.

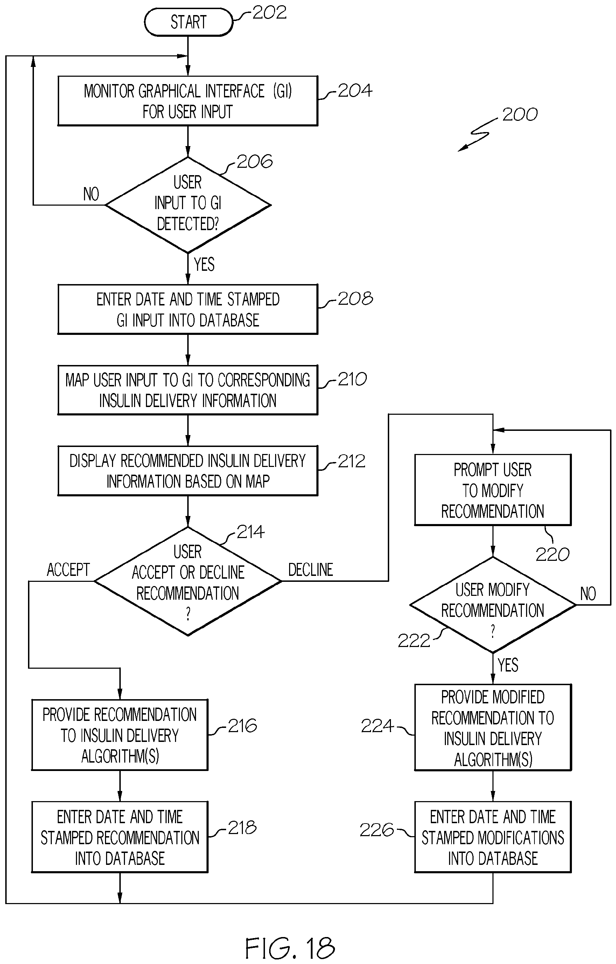

Referring now to FIG. 18, a flowchart is shown of one illustrative embodiment of the bolus assessment/adjustment algorithm 200 for determining drug administration information based on patient input, e.g., meal intake information, to the graphical interface 20. The software algorithm 200 will be described as being executed by the processor 14 of the electronic device 12 (see FIG. 1). The algorithm 200 begins at step 202, and at step 204 the processor 14 is operable to monitor the graphical interface (GI) 20 for patient input of meal intake information. The graphical interface 20 may take the form of any one or any combination of the example graphical interfaces illustrated and described herein with respect to FIGS. 7-12, or may alternatively take some other form providing for the patient input of meal-related carbohydrate content and expected speed of overall glucose absorption from the meal by the patient.

Following step 204, the algorithm 200 advances to step 206 where the processor 14 is operable to determine whether a complete user input to the graphical interface 20 has been detected. In embodiments having a single-input graphical user interface, for example, the processor 14 may be operable at step 206 to determine when a complete user input to the graphical interface 20 has occurred when the patient has selected a single input to the graphical interface 20. In embodiments having a multiple-input graphical interface 20, on the other hand, the processor 14 may be operable to determine when a complete user input to the graphical interface 20 has occurred when the user has selected all user-selectable inputs to the graphical interface 20. In any case, if at step 206 the processor has not detected a complete patient input to the graphical interface 20, execution of the algorithm 200 loops back to execute step 204. If, on the other hand, the processor 14 detects at step 206 that a complete patient input to the graphical interface 20 has occurred, algorithm execution advances to step 208 where the processor 14 is operable to time and date stamp the graphical interface 20 input and to enter the date and time stamped graphical interface 20 input into a database contained within the memory or data storage unit 16 and/or 34. Steps 204 and 206 may illustratively further include a timeout mechanism configured to direct the algorithm 200 to a specified step or state if the user does not provide a complete user input to the graphical interface 20 within a specified time period.

In the illustrated embodiment, the algorithm 200 is arranged with the expectation that the patient will enter the meal-related information just prior to ingesting the meal so that the date and time stamp is generally indicative of the date and time that the meal is actually consumed. Step 208 may illustratively be modified to further provide the patient with the ability to modify the time and/or date associated with the date and time stamped graphical interface 20 entry prior to entering this information into the database. This optional feature provides the user with the ability to enter the meal intake information into the graphical interface 20 after ingesting the meal, and to then alter the time and/or date of the date stamp from the current time and/or date to reflect an actual or estimated previous time and/or date that the meal was ingested. In this manner, for example, meal compensation boluses may be determined and administered or recommended after the meal has been ingested. This optional feature also provides the patient with the ability to enter the meal intake information into the graphical interface 20 before ingesting the meal, e.g., sufficiently before the meal so that the date and/or time stamp of step 208 would generally not be indicative of the actual time and/or date that the corresponding meal is consumed, and to then alter the time and/or date stamp from the current time and/or date to reflect an estimated future time and/or date that the meal will likely be ingested. It should be understood, however, that in either case the algorithm 200 should further include one or more steps that allow the processor 14 to appropriately modify the user-entered input to the graphical interface 20 for use in determining the meal compensation bolus so that the speed of overall glucose absorption from the meal by the patient takes into account the time elapsed between ingesting the meal and subsequently entering the meal-related user input to the graphical interface 20, or the time delay between entering the meal-related user input the graphical interface 20 and subsequently ingesting the meal. Inclusion of such one or more steps would be a mechanical exercise for a skilled programmer.

Although not shown in FIG. 18, the algorithm 200 or another independently executing algorithm may further include one or more steps that allow the user to modify previously entered meal-related information and/or associated time and/or date stamp information, or to append new and/or perhaps more accurate information onto the previously entered meal-related information and/or associated time and/or date stamp information. This optional feature provides the patient with the ability to modify such data, such as in cases where the meal-related information was entered prior to or during ingestion of the meal, to subsequently reflect any deviations in actual meal ingestion from that which was expected or estimated at the time the information was entered. For example, a scheduled meal may be skipped or delayed, more or less of the meal may have actually been consumed as compared with what was previously estimated, and/or the composition of the meal may have varied from what was previously estimated.

Following step 208, the processor 14 is operable at step 210 to map the user (patient) input of meal intake information to the graphical interface 20 to corresponding insulin delivery information. The memory or data storage unit 16 and/or 34 illustratively has stored therein a map correlating the patient-entered meal information to insulin delivery amount(s) and time(s), one embodiment of which is illustrated and described herein with respect to FIG. 16. It will be understood, however, that the processor 14 may alternatively or additionally use different table axes values that are consistent with carbohydrate content and expected speed of overall glucose absorption from the meal by the patient, and/or use one or more other conventional mapping techniques for mapping the user-specified meal intake information to corresponding insulin bolus delivery information. While the insulin bolus delivery information was described with respect to FIG. 16 as comprising a meal compensation bolus including any one or more of total number, X, of insulin boluses to be administered to the user, an amount or quantity, Y, of each of the number of insulin boluses to be administered (e.g., in international units), a time, $\Delta T$, between each of the insulin boluses to be administered and a time, I, that a first one of the number of insulin boluses will be administered, it will further be understood that the insulin bolus delivery information may alternatively or additionally include one or more correction bolus amounts, i.e., insulin bolus amounts not related to meals, and in any case may include more or less information than that illustrated in FIG. 16.

Execution of the algorithm 200 advances from step 210 to step 212 where the processor 14 is operable, in the illustrated embodiment, to control the display unit 20 and/or display unit 38 to display, in the form of an insulin bolus recommendation, at least some of the insulin delivery information determined at step 210. Thereafter at step 214, the processor 14 is operable to determine whether the user accepts or declines the insulin bolus recommendation displayed at step 212. In one exemplary embodiment, the processor 14 is operable to execute step 214 by first displaying, along with the insulin bolus recommendation, graphical "accept" and "decline" indicators that are selectable by the user, and then monitoring such indictors to determine which of the two the user selects. In an alternative embodiment, "accept" and "decline" buttons or keys may form part of the input device 18 and/or 36, and the processor 14 is operable in this embodiment to execute step 214 by monitoring such buttons or keys to determine which of the two the user selects. Those skilled in the art will recognize other conventional techniques for accomplishing step 214, and any such other conventional techniques are contemplated by this disclosure. Step 214 may illustratively further include a timeout mechanism configured to direct the algorithm 200 to a specified step or state if the user does not accept or decline the recommendation displayed at step 212. In any case, if the processor 14 determines at step 214 that the user accepts the insulin bolus recommendation displayed at step 212, the processor 14 is thereafter operable at step 216 to provide the recommended insulin bolus information to one or more insulin delivery algorithms, e.g., the bolus determination algorithm 156 of FIG. 17. In the illustrative embodiment, the bolus determination algorithm 156 after receiving the insulin recommendation, will then dispense at the appropriate therapy time the commanded insulin based on pump capabilities and user settings. For example, some pumps dispense insulin in rapid pulses and some do it as one injection. Using these capability insulin can be released in one embodiment according to a profile. Thereafter at step 218, the processor 14 is operable to date and time stamp the recommended insulin bolus information and enter the date and time stamped recommended insulin bolus information into a database contained within the memory or data storage unit 16 and/or 34.

If, at step 214, the processor 14 determines that the patient rejects the insulin bolus recommendation displayed at step 212, the processor 14 is thereafter operable at step 220 to prompt the user to modify the recommended insulin bolus information. In one exemplary embodiment, the processor 14 is operable to execute step 220 by displaying the insulin bolus recommendation in a manner that allows the patient to modify any of the recommended insulin bolus information via the graphical interface 20, input device 18 and/or 36, or via some other conventional data input device, and to also display a graphical "accept changes" indicator that is selectable by the user when modifications to the recommended insulin bolus information are complete. The processor 14 is then operable at step 222 to monitor the "accept changes" indicator. Until the user selects the "accept changes" indicator at step 222, the algorithm 200 loops back to execute step 220. The algorithm may further illustratively include one or more conventional steps (not shown) that allow the algorithm 200 to continue past step 222 if the user does not select the "accept changes" indicator within a specified time period. In any case, when the processor 14 determines at step 222 that the user has selected the "accept changes" indicator, the processor 14 is thereafter operable at step 224 to provide the modified insulin bolus information to one or more insulin delivery algorithms, e.g., the bolus determination algorithm 156 of FIG. 17. Thereafter at step 226, the processor 14 is operable to date and time stamp the modified insulin bolus information and enter the date and time stamped modified insulin bolus information into a database contained within the memory or data storage unit 16 and/or 34. In an alternate embodiment, steps 216 - 224 of the algorithm 200 may be modified in a conventional manner to allow the user to manually override the recommended insulin bolus information by manually administering one or more insulin boluses. In this embodiment, however, it is desirable to allow the patient to enter into the database, at steps 218 and 226, date and time stamped information relating to the manual administration of the one or more insulin boluses, e.g., number, type, quantity and/or timing of the one or more insulin boluses. In any case, the execution of the algorithm 200 loops from either of steps 218 and 226 back to step 204.

In an alternative embodiment of the algorithm 200, steps 212 -216 and 220-224 may be modified in a conventional manner to cause the processor 14 to control, under the direction of one or more insulin delivery algorithms, automatic administration of one or more insulin boluses to the user according to the insulin delivery information determined at step 210. In this embodiment, the insulin delivery information determined at step 210 is therefore not displayed or otherwise offered to the user as an insulin bolus recommendation, but can instead be automatically administered or otherwise delivered to the user via a conventional electronically controlled insulin delivery device, e.g., implantable, subcutaneous, transcutaneous and/or transdermal insulin infusion pump. Collected information may further be synchronized with a centralized data base wherein all patient records are maintained. The information may be exchanged using standardized data exchange protocol such as HL7, CDISC. The system also envisions using proprietary protocol for data exchange.)

The graphical user interface examples illustrated herein for determining drug administration information, based on patient-related input information via a graphical interface, have been presented in the context of supplying the graphical interface with meal intake information from which insulin delivery information is determined. It will be understood that similar graphical interfaces may alternatively or additionally be developed based wholly or in part based on one or more other patient-related events or conditions, such as one or more external influences and/or various physiological mechanisms associated with the patient. Examples include, but are not limited to, considerations such as explicit or implicit one or two-dimensional indicators of exercise, stress, illness, menstrual cycle and/or the like. Further examples are provided in commonly assigned and co-pending U.S. Patent Application US 2007/0179434 A1. Those skilled in the art will recognize examples of other graphical user interfaces that may be developed based on one or more other external influences and/or various physiological mechanisms associated with the user, and any such other examples are contemplated by this disclosure. In any case, the processor 14 is illustratively operable with any such graphical user interfaces to date and time stamp event occurrences, and may additionally allow the time and date stamp to be altered to identify that the one or more other external influences and/or various physiological mechanisms associated with the user occurred in the past or is expected to occur in the future. This feature also illustratively allows for the capability of providing the user with reminders of start/stop times of upcoming (e.g., scheduled) events in order to increase accuracy of the system and provide for an increased level of event compliance.

Whether any one or more of the graphical interfaces illustrated and described herein are suitable for use by a patient will depend, at least in part, upon that patient's personal habits. For example, whether a graphical interface correlating meal intake information to meal-related insulin delivery information as described herein is suitable for use by a patient will depend, at least in part, upon the dietary habits of that patient. It is accordingly desirable to develop one or more appropriate graphical interfaces for any patient based on that patient's habits and taking into account the patient's suitability for the use of any such graphical interface based on such habits. In order to reduce the amount of input provided by the user to the system 10 without jeopardizing the overall level of glucose control, regularities in the patient's habits are exploited. Thus, for example, whether or not a meal-related graphical interface of the type illustrated and described herein is suitable for use by an individual depends generally on the ability to simplify the variability of meals or snacks in relation to their glycemic consequences by exploiting predictabilities in the individual's eating habits.

While the invention has been illustrated and described in detail in the foregoing drawings and description, the same is to be considered as illustrative and not restrictive in character, it being understood that only illustrative embodiments thereof have been shown and described.

## Claims

1. A method of developing a patient specific graphical interface for a therapy system that an individual patient may use to input information which characterizes at least one patient event or condition and from which therapy information can be determined, comprising:

   providing a patient model that is tailored to the physiology of the individual patient and configured to simulate a physiological response in the individual patient to the at least one patient event or condition,
   collecting patient-specific information over time that relates to actual occurrences of the at least one patient event or condition, and **characterized by**,
   providing the patient specific graphical interface with a solution space that is based on the patient model and on ranges of inputs defined by the collected patient-specific information and which provides a relationship between the inputs and associated acceptable limits of the inputs to regulate a physiological response of the patient to a desired target response, and in which the inputs each characterizes the at least one patient event or condition and are mapped to corresponding therapy information for regulating the physiological response of the patient to the desired target response for the characterized at least one patient event or condition.

2. The method of claim 1 wherein the therapy information comprises drug therapy information corresponding to one or more drugs to be administered to the patient.

3. The method of claim 1 wherein the therapy information comprises suggested carbohydrate intake information corresponding to a recommendation to ingest carbohydrates.

4. The method of claim 1 wherein the therapy information comprises suggested exercise information corresponding to a recommendation to undertake exercise.

5. The method of claim 1 wherein the therapy information comprises a recommendation to consult a physician.

6. The method of claim 1 further comprising using the graphical interface to map the patient input of the information which characterizes the at least one patient event or condition to corresponding therapy information.

7. The method of claim 6 wherein the corresponding therapy information comprises drug administration information.

8. A system for developing a patient specific graphical interface for a therapy system that an individual patient may use to input information which characterizes at least one patient event or condition and from which therapy information can be determined, comprising:

   a database having stored therein a patient model that is tailored to the physiology of the individual patient and configured to simulate the patient's physiological response to the at least one patient event or condition,

   a first memory configured to store therein patient-specific information over time that relates to actual occurrences of the at least one patient event or condition, and **characterized by**,
   a patient specific graphical interface having a solution space that is based on the patient model and on ranges of inputs defined by the collected patient-specific information and which provides a relationship between the inputs and associated acceptable limits of the inputs to regulate a physiological response of the patient to a desired target response, and in which the inputs each characterizes the at least one patient event or condition and are mapped to corresponding therapy information for regulating the physiological response of the patient to the desired target response for the characterized at least one patient event or condition.

9. The system of claim 8 further comprising a processor having access to a second memory that has stored therein instructions that are executable by the processor to process the input from the patient to the graphical interface of the information which characterizes the at least one patient and produce the corresponding therapy information.

10. The system of claim 9 further comprising a display unit, wherein the second memory further has stored therein instructions that are executable by the processor to control the display unit to display the corresponding therapy information.

11. The system of claim 10 further comprising a manually actuatable drug administration device, wherein the corresponding therapy information comprises at least one drug quantity, and wherein the second memory further has stored therein instructions that are executable by the processor to control the display unit to display the at least one drug quantity that may be administered by the patient using the manually actuatable drug administration device.

12. The system of claim 11 further comprising a blood glucose sensor configured to measure a blood glucose level of the patient and produce a corresponding blood glucose value, and wherein the second memory further has stored therein instructions that are executable by the processor to determine the at least one drug quantity further based on the blood glucose value.

13. The system of claim 12 further comprising an electronically controllable drug administration device configured to administer at least one drug to the patient, wherein the corresponding therapy information comprises at least one drug quantity, and wherein the second memory further has stored therein instructions that are executable by the processor to control the electronically controllable drug administration device to administer the at least one drug quantity to the patient.

14. The system of claim 8 wherein the graphical interface is configured to map patient input of at least two parameters that characterize the at least one patient or condition to the corresponding therapy information.

**Patentansprüche**

1. Verfahren zum Entwickeln einer patientenspezifischen grafischen Schnittstelle für ein Therapiesystem, das ein individueller Patient zum Eingeben von Informationen verwenden kann, die mindestens ein Patientenereignis oder einen Patientenzustand charakterisieren und woraus Therapieinformationen bestimmt werden können, umfassend:

Bereitstellen eines Patientenmodells, das auf die Physiologie des individuellen Patienten maßgeschneidert ist und konfiguriert ist, um eine physiologische Reaktion in dem individuellen Patienten auf das mindestens eine Patientenereignis oder den Patientenzustand zu simulieren,
Sammeln von patientenspezifischen Informationen über die Zeit, welche tatsächliche Vorkommnisse des mindestens einen Patientenereignisses oder Patientenzustands betreffen, und **gekennzeichnet durch**:

Bereitstellen eines Lösungsraums für die patientenspezifische grafische Schnittstelle, der auf dem Patientenmodell und auf Bereichen von Eingaben basiert, die **durch** die gesammelten patientenspezifischen Informationen definiert sind, und der eine Beziehung zwischen den Eingaben und dazugehörigen akzeptablen Grenzen der Eingaben bereitstellt, um eine physiologische Reaktion des Patienten auf eine gewünschte Zielreaktion zu regulieren, und wobei die Eingaben jeweils das mindestens eine Patientenereignis oder den Patientenzustand charakterisieren und auf entsprechende Therapieinformationen abgebildet werden, um die physiologische Reaktion des Patienten auf die gewünschte Zielreaktion für das charakterisierte mindestens eine Patientenereignis oder den Patientenzustand zu regulieren.

2. Verfahren nach Anspruch 1, wobei die Therapieinformationen Arzneimitteltherapieinformationen umfassen, die einem oder mehreren Arzneimitteln entsprechen, die dem Patienten zu verabreichen sind.

3. Verfahren nach Anspruch 1, wobei die Therapieinformationen vorgeschlagene Kohlenhydrateinnahmeinformationen umfassen, die einer Empfehlung zur Aufnahme von Kohlenhydraten entsprechen.

4. Verfahren nach Anspruch 1, wobei die Therapieinformationen vorgeschlagene Leibungsübungsinformationen umfassen, die einer Empfehlung entsprechen, Leibesübungen durchzuführen.

5. Verfahren nach Anspruch 1, wobei die Therapieinformationen eine Empfehlung umfassen, einen Arzt aufzusuchen.

**6.** Verfahren nach Anspruch 1, ferner umfassend die Verwendung der grafischen Schnittstelle, um die Patienteneingabe der Informationen, die das mindestens eine Patientenereignis oder den Pateintenzustand charakterisieren, auf entsprechende Therapieinformationen abzubilden.

**7.** Verfahren nach Anspruch 6, wobei die entsprechenden Therapieinformationen Arzneimittelverabreichungsinformationen umfassen.

**8.** System zum Entwickeln einer patientenspezifischen grafischen Schnittstelle für ein Therapiesystem, das ein individueller Patient zum Eingeben von Informationen verwenden kann, die mindestens ein Patientenereignis oder einen Patientenzustand charakterisieren und woraus Therapieinformationen bestimmt werden können, umfassend:

eine Datenbank, in der ein Patientenmodell gespeichert ist, das auf die Physiologie des individuellen Patienten maßgeschneidert ist und konfiguriert ist, um die physiologische Reaktion des Patienten auf das mindestens eine Patientenereignis oder den Patientenzustand zu simulieren,
einen ersten Speicher, der konfiguriert ist, um darin patientenspezifische Informationen über die Zeit zu sammeln, welche tatsächliche Vorkommnisse des mindestens einen Patientenereignisses oder - Patientenzustands betreffen, und **gekennzeichnet durch**:

eine patientenspezifische grafische Schnittstelle mit einem Lösungsraum, der auf dem Patientenmodell und auf Bereichen von Eingaben basiert, die **durch** die gesammelten patientenspezifischen Informationen definiert sind, und der eine Beziehung zwischen den Eingaben und dazugehörigen akzeptablen Grenzen der Eingaben bereitstellt, um eine physiologische Reaktion des Patienten auf eine gewünschte Zielreaktion zu regulieren, und wobei die Eingaben jeweils das mindestens eine Patientenereignis oder den Patientenzustand charakterisieren und auf entsprechende Therapieinformationen abgebildet sind, um die physiologische Reaktion des Patienten auf die gewünschte Zielreaktion für das charakterisierte mindestens eine Patientenereignis oder den Patientenzustand zu regulieren.

**9.** System nach Anspruch 8, ferner umfassend einen Prozessor mit Zugriff auf einen zweiten Speicher, in dem Anweisungen gespeichert sind, die durch den Prozessor ausführbar sind, um die von dem Patienten vorgenommenen Eingaben der Informationen an die grafische Schnittstelle zu verarbeiten, welche den mindestens einen Patienten charakterisieren und die entsprechenden Therapieinformationen produzieren.

**10.** System nach Anspruch 9, ferner umfassend eine Anzeigeeinheit, in der der zweite Speicher ferner Anweisungen gespeichert hat, die durch den Prozessor ausführbar sind, um die Anzeigeeinheit zu steuern, die entsprechenden Therapieinformationen anzuzeigen.

**11.** System nach Anspruch 10, ferner umfassend eine manuell betätigbare Arzneimittelverabreichungsvorrichtung, wobei die entsprechenden Therapieinformationen mindestens eine Arzneimittelmenge umfassen, und wobei der zweite Speicher ferner Anweisungen darin gespeichert hat, die durch den Prozessor ausführbar sind, um die Anzeigeeinheit zu steuern, um die mindestens eine Arzneimittelmenge anzuzeigen, die dem Patienten unter Verwendung der manuell betätigbaren Arzneimittelverabreichungsvorrichtung verabreicht werden darf.

**12.** System nach Anspruch 11, ferner umfassend einen Blutglukosesensor, der zum Messen eines Blutglukosespiegels des Patienten und zum Produzieren eines entsprechenden Blutglukosewerts konfiguriert ist, und wobei der zweite Speicher ferner darin Anweisungen gespeichert hat, die durch den Prozessor ausführbar sind, um die mindestens eine Arzneimittelmenge ferner basierend auf dem Blutglukosewert zu bestimmen.

**13.** System nach Anspruch 12, ferner umfassend eine elektronisch steuerbare Arzneimittelverabreichungsvorrichtung, die zum Verabreichen von mindestens einem Arzneimittel an den Patienten konfiguriert ist, wobei die entsprechenden Therapieinformationen mindestens eine Arzneimittelmenge umfassen, und wobei der zweite Speicher ferner darin Anweisungen gespeichert hat, die durch den Prozessor ausführbar sind, um die elektronisch steuerbare Arzneimittelverabreichungsvorrichtung zu steuern, um die mindestens eine Arzneimittelmenge an den Patienten zu verabreichen.

**14.** System nach Anspruch 8, wobei die grafische Schnittstelle konfiguriert ist, um die Patienteneingabe von mindestens zwei Parametern, die den mindestens einen Patienten oder Zustand charakterisieren, auf die entsprechenden Therapieinformationen abzubilden.

**Revendications**

1. Procédé de développement d'une interface graphique spécifique à un patient pour un système thérapeutique qu'un patient individuel peut utiliser pour entrer des informations qui caractérisent au moins un événement ou un état de patient et à partir duquel des informations thérapeutiques peuvent être déterminées, comprenant les étapes consistant à :

fournir un modèle de patient qui est adapté à la physiologie du patient individuel et est configuré pour simuler une réponse physiologique du patient individuel au au moins un événement ou état de patient,
collecter au fil du temps des informations spécifiques au patient qui concernent des survenues effectives du au moins un événement ou état de patient, et **caractérisé par** les étapes consistant à,
munir l'interface graphique spécifique au patient d'un espace de solution qui est basé sur le modèle de patient et sur des plages d'entrées définies grâce aux informations spécifiques au patient collectées et qui fournit une relation entre les entrées et des limites acceptables associées desdites entrées afin de réguler une réponse physiologique du patient par rapport à une réponse cible souhaitée, et dans laquelle les entrées caractérisent respectivement le au moins un événement ou état de patient et sont mises en correspondance avec des informations thérapeutiques correspondantes afin de réguler la réponse physiologique du patient par rapport à la réponse cible souhaitée pour le au moins un événement ou état de patient caractérisé.

2. Procédé selon la revendication 1, dans lequel les informations thérapeutiques comprennent des informations pharmacothérapeutiques correspondant à un ou plusieurs médicament(s) à administrer au patient.

3. Procédé selon la revendication 1, dans lequel les informations thérapeutiques comprennent des informations de consommation d'hydrates de carbone correspondant à une recommandation d'ingestion d'hydrates de carbone.

4. Procédé selon la revendication 1, dans lequel les informations thérapeutiques comprennent des informations d'exercice suggérées correspondant à une recommandation de pratique d'exercice physique.

5. Procédé selon la revendication 1, dans lequel les informations thérapeutiques comprennent une recommandation de consultation d'un médecin.

6. Procédé selon la revendication 1, comprenant en outre une étape consistant à utiliser l'interface graphique pour mettre en correspondance avec des informations thérapeutiques correspondantes l'entrée, par le patient, de l'information qui caractérise le au moins un événement ou état de patient.

7. Procédé selon la revendication 6, dans lequel les informations thérapeutiques correspondantes comprennent des informations d'administration de médicament.

8. Système permettant de développer une interface graphique spécifique au patient pour un système thérapeutique qu'un patient individuel peut utiliser pour entrer des informations qui caractérisent au moins un événement ou état de patient et à partir desquelles des informations thérapeutiques peuvent être déterminées, comprenant :

une base de données présentant, stocké en son sein, un modèle de patient qui est adapté à la physiologie du patient individuel et est configuré pour simuler la réponse physiologique du patient au au moins un événement ou état de patient,
une première mémoire configurée pour stocker en son sein au fil du temps des informations spécifiques au patient qui concernent des survenues effectives du au moins un événement ou état de patient, et **caractérisé par**,
une interface graphique spécifique au patient présentant un espace de solution qui est basé sur le modèle de patient et sur des plages d'entrées définies par les informations spécifiques au patient collectées et qui fournit une relation entre les entrées et des limites acceptables associées des entrées afin de réguler une réponse physiologique du patient par rapport à une réponse cible souhaitée, et dans lequel les entrées caractérisent respectivement le au moins un événement ou état de patient et sont mises en correspondance avec des informations thérapeutiques correspondantes afin de réguler la réponse physiologique du patient par rapport à la réponse cible souhaitée pour le au moins un événement ou état de patient caractérisé.

9. Système selon la revendication 8, comprenant en outre un processeur ayant accès à une deuxième mémoire qui présente, stockées en son sein, des instructions qui peuvent être exécutées par ledit processeur afin de traiter l'entrée, par le patient, dans l'interface graphique, des informations qui caractérisent le au moins un patient et afin de produire les informations thérapeutiques correspondantes.

**10.** Système selon la revendication 9, comprenant en outre une unité d'affichage, dans lequel la deuxième mémoire présente en outre, stockées en son sein, des instructions qui peuvent être exécutées par le processeur afin de commander l'unité d'affichage pour qu'elle affiche les informations thérapeutiques correspondantes.

**11.** Système selon la revendication 10, comprenant en outre un dispositif d'administration de médicament pouvant être actionné manuellement, dans lequel les informations thérapeutiques correspondantes comprennent au moins une quantité de médicament, et dans lequel la deuxième mémoire présente en outre, stockées en son sein, des instructions qui peuvent être exécutées par le processeur afin de commander l'unité d'affichage pour qu'elle affiche la au moins une quantité de médicament qui peut être administrée par le patient en utilisant le dispositif d'administration de médicament pouvant être actionné manuellement.

**12.** Système selon la revendication 11, comprenant en outre un capteur de glycémie configuré pour mesurer un niveau de glycémie du patient et produire une valeur de glycémie correspondante, et dans lequel la deuxième mémoire présente en outre, stockées en son sein, des instructions qui peuvent être exécutées par le processeur afin de déterminer la au moins une quantité de médicament en se basant en outre sur la valeur de glycémie.

**13.** Système selon la revendication 12, comprenant en outre un dispositif d'administration de médicament pouvant être commandé de manière électronique et configuré pour administrer au moins un médicament au patient, dans lequel les informations thérapeutiques correspondantes comprennent au moins une quantité de médicament, et dans lequel la deuxième mémoire présente en outre, stockées en son sein, des instructions qui peuvent être exécutées par le processeur afin de commander le dispositif d'administration de médicament pouvant être commandé de manière électronique afin d'administrer la au moins une quantité de médicament au patient.

**14.** Système selon la revendication 8, dans lequel l'interface graphique est configurée pour mettre en correspondance avec les informations thérapeutiques correspondantes une entrée, par le patient, d'au moins deux paramètres qui caractérisent le au moins un événement ou état de patient.

EP 2 170 158 B1

FIG. 1

FIG. 2

MEALS ⟋72

80⟍ OTHER(S)

EXERCISE ⟋74

GLUCO-REGULATORY
MODEL

⟋70

PATIENT-SPECIFIC
PHYSIOLOGY

78⟍ ILLNESS

STRESS ⟋76

GLUCOSE-LOWERING
DRUGS ⟋75

FIG. 3

$^r$SOURCE1 $-$ $^r$SINK1

$Q_B$ , $C_{Bi}$

CAPILLARY BLOOD

$V_B$ , $C_{BO}$

$Q_B$ , $C_{BO}$

85⟍

INTERSTITIAL FLUID

$V_I$ , $C_I$

PA

$^r$SINK2 $+$ $^r$SOURCE2

FIG. 4

FIG. 5

FIG.6

FIG. 7

FIG. 8

MEAL INTAKE INFORMATION        DATE/TIME

FAT AMT

PROTEIN AMT

CARB AMT

SM        MED        LG

}114        }20

MEAL SIZE

FIG. 9

MEAL INTAKE INFORMATION        DATE/TIME

FAT AMT

PROTEIN AMT

CARB AMT

SMALLER    NORM.    LARGER
THAN NORM.        THAN NORM.

}116        }20

MEAL SIZE

FIG. 10

DATE/TIME

MEAL INTAKE INFORMATION

TOTAL
GLYCEMIC
INDEX

75

50

50 100 150

CARBS (gr)

118

20

FIG. 11

DATE/TIME

MEAL INTAKE INFORMATION

FAST

MEAL
DURATION

MED

SLOW

SM MED LG

MEAL SIZE

120

20

FIG. 12

$\eta^S$

130

FINAL GRID

$\Delta\eta^S$

110

FIG. 13

$\eta^A$

$\Delta\eta^A$

ACCEPTABLE
SOLUTION SPACE

$I_0^M$

SOLVING LOW
BG BOUNDARY

138

136

FIG. 14

SOLVING HIGH
BG BOUNDARY

134

$\eta_{A/S}$

FOR SELECTED MEAL TYPE

MEAL AMOUNT CHANGING
WITH MEAL SPEED FIXED

PARAMETER
AXIS

140

$\eta_4$

$\eta_3$

FIG. 15

$\eta_2$

$\eta_1$

USER

LEVEL 1

LEVEL 2

LEVEL 3

MEAL SIZE

|  | SMALL | MEDIUM | LARGE |
|---|---|---|---|
| FAST | TOTAL NO. OF BOLUSES = X<br>AMT OF EACH BOLUS = Y<br>TIME BETWEEN BOLUSES =$\Delta$T<br>TIME OF FIRST BOLUS = I | ● | ● |
| MEDIUM | ● |  |  |
| SLOW | ● |  |  |

MEAL DURATION

130

FIG. 16

FIG. 17

PATIENT — 152

bG

INSULIN DELIVERY DEVICE — 154

bG SENSOR — 158

MEALS — 72
EXERCISE — 74
ILLNESS — 78
STRESS — 76
OTHER(S) — 80

12

BOLUS DETERMINATION ALGORITHM — 156

bG SENSOR — 160

BOLUS ASSESSMENT ALGORITHM — 200

ELECTRONIC DEVICE

150

GRAPHICAL INTERFACE — 20

EP 2 170 158 B1

START ~202

MONITOR GRAPHICAL INTERFACE (GI) FOR USER INPUT ~204

~200

USER INPUT TO GI DETECTED? ~206

NO

YES

ENTER DATE AND TIME STAMPED GI INPUT INTO DATABASE ~208

MAP USER INPUT TO GI TO CORRESPONDING INSULIN DELIVERY INFORMATION ~210

DISPLAY RECOMMENDED INSULIN DELIVERY INFORMATION BASED ON MAP ~212

PROMPT USER TO MODIFY RECOMMENDATION

220

USER ACCEPT OR DECLINE RECOMMENDATION? ~214

ACCEPT

DECLINE

USER MODIFY RECOMMENDATION? 222

NO

YES

PROVIDE RECOMMENDATION TO INSULIN DELIVERY ALGORITHM(S) ~216

PROVIDE MODIFIED RECOMMENDATION TO INSULIN DELIVERY ALGORITHM(S) 224

ENTER DATE AND TIME STAMPED RECOMMENDATION INTO DATABASE ~218

ENTER DATE AND TIME STAMPED MODIFICATIONS INTO DATABASE 226

FIG. 18

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 03063684 A2
- WO 2009002621A2 A
- WO 2009002620A1 A
- US 20070179434 A1

**Non-patent literature cited in the description**

- **LEON SHARGEL ; ANDREW B.C. YU.** *Applied Biopharmaceutics & Pharmacokinetics*
- **MEHDI BOROUJERDI.** *Pharmacokinetics, Principles and Applications*
- **JOHN THOMAS SORENSEN.** A physiologic model of glucose metabolism in man and its use to design and assess improved insulin therapies for diabetes. *PhD, MIT,* 1985
- **PER-OLOF ASTRAND ; KAARE RODAHL ; HANS A. DAHL ; SIGMUND B. STROMME.** Textbook of Work Physiology, Physiological bases of Exercise
- **MOTOAKI SHICHIRI.** *Artificial Endocrine Pancreas*
- **RICHARD BERGMAN ; JENNIFER C. LOVEJOY.** The minimal model approach and determinants of glucose tolerance. *Penington Center Nutrition Series,* vol. 7
- **MARCO PAOLO DERIGHETTI.** Feedback control in Anaesthesia. *PhD Swiss Federal Institute of Technology*
- **KWON ; BRUCKSTEIN ; KAILATH.** Stabilizing state feedback design via the moving horizon method. *Intl. Journal of Control,* 1983, vol. 37, 631-643
- **GARCIA ; PRETT ; MORARI.** Model predictive control: theory and practice. *Automatica,* 1989, vol. 25, 335-348
- **ROMAN HOVORKA ; VALENTINA CANONICO ; LUDOVIC J CHASSIN ; ULRICH HAUETER ; MASSIMO MASSI-BENEDETTI ; MARCO ORSINI FEDERICI ; THOMAS R PIEBER ; HELGA C SCHALLER ; LUKAS SCHAUPP ; THOMAS VERING.** Nonlinear model predictive control of glucose concentration in subjects with type 1 diabetes. *Physiol. Meas.,* 2004, vol. 25, 905-920